# EUROPEAN PATENT SPECIFICATION

(11) **EP 1 097 155 B1**
(45) Date of publication and mention of the grant of the patent: **18.09.2002**
(21) Application number: 99932363.7
(22) Date of filing: 09.07.1999
(51) Int. Cl.: C07D 493/04

(54) **PHOTOCHROMIC SIX-MEMBERED HETEROCYCLIC-FUSED NAPHTHOPYRANS**
PHOTOCHROME SECHSGLIEDRIGE HETEROCYCLISCH KONDENSIERTE NAPHTHOPYRANE
NOUVEAUX NAPHTOPYRANES PHOTOCHROMIQUES CONDENSES AU NOYAU HETEROCYCLIQUE A SIX ELEMENTS

(30) Priority: 10.07.1998 US 114102; 19.03.1999 US 273086
(43) Date of publication of application: 09.05.2001
(73) Proprietor: TRANSITIONS OPTICAL, INC., Pinellas Park, FL 33782 (US)
(72) Inventor: KUMAR, Anil, Pittsburgh, PA 15239 (US)
(74) Representative: Sternagel, Fleischer, Godemeyer & Partner Patentanwälte
(86) International application number: PCT/US99/15524
(87) International publication number: WO 00/002883

(56) References cited:
- WO-A-97/15565
- WO-A-97/21698
- WO-A-99/28323
- US-A- 5 458 814
- US-A- 5 565 147
- US-A- 5 674 432
- US-A- 5 679 805

## Description

### CROSS REFERENCE TO RELATED APPLICATIONS

This application is a continuation-in-part of U.S. Serial No. 09/114,102, filed July 10, 1998

### DESCRIPTION OF THE INVENTION

The present invention relates to certain novel naphthopyran compounds. More particularly, this invention relates to novel photochromic naphthopyran compounds having a six-membered heterocyclic ring fused to the naphtho portion of the molecule and to compositions and articles containing such novel naphthopyran compounds. When exposed to electromagnetic radiation containing ultraviolet rays, such as the ultraviolet radiation in sunlight or the light of a mercury lamp, many photochromic compounds exhibit a reversible change in color. When the ultraviolet radiation is discontinued, such a photochromic compound will return to its original color or colorless state.

Various classes of photochromic compounds have been synthesized and suggested for use in applications in which a sunlight-induced reversible color change or darkening is desired. U.S. Patent 3,567,605 (Becker) describes a series of pyran derivatives, including certain benzopyrans and naphthopyrans. These compounds are described as derivatives of chromene and are reported to undergo a color change, e.g., from colorless to yellow-orange, on irradiation by ultraviolet light at temperatures below about -30°C. Irradiation of the compounds with visible light or upon raising the temperature to above about 0°C is reported to reverse the coloration to a colorless state.

U.S. Patent 5,066,818 describes various 3,3-diaryl-3H-naphtho[2,1-b]pyrans as having desirable photochromic properties, i.e., high colorability and acceptable fade, for ophthalmic and other applications. Also disclosed by way of comparative example in the '818 patent are the isomeric 2,2-diaryl-2H-naphtho[1,2-b]pyrans, which are reported to require unacceptably long periods of time to fade after activation.

U.S. Patent 3,627,690 describes photochromic 2,2-di-substituted-2H-naphtho[1,2-b]pyran compositions containing minor amounts of either a base or weak-to-moderate strength acid. The addition of either an acid or base to the naphthopyran composition is reported to increase the fade rate of the colored naphthopyrans, thereby making them useful in eye protection applications such as sunglasses. It is reported therein further that the fade rate of 2H-naphtho-[1,2-b]pyrans without the aforementioned additives ranges from several hours to many days to reach complete reversion.

U.S. Patent 4,818,096 discloses purple/blue coloring photochromic benzo- or naphthopyrans having at the position alpha to the oxygen of the pyran ring a phenyl group having a nitrogen containing substituent in the ortho or para positions. U.S. Patent 5,645,767 describes novel photochromic indeno-fused 2H-naphtho[1,2-b]pyran compounds, the 2,1-positions of the indeno group being fused to the f side of the naphthopyran.

U.S. Patent 5,458,814 discloses photochromic 2H-naphtho[1,2-b]pyran compounds having certain substituents at the number 5 and 6 carbon atoms of the naphtho portion of the naphthopyran and at the 2-position of the pyran ring. These compounds have an acceptable fade rate in addition to a high activated intensity and a high coloration rate.

The present invention relates to novel substituted naphtho[1,2-b]pyran compounds having a substituted or unsubstituted six-membered heterocyclic group fused to the f side of the naphtho portion of the naphthopyran with certain substituents at the position ortho to the oxygen atom of the naphthopyran, which compounds may be represented by graphic formula I. These compounds have demonstrated an acceptable fade rate without the addition of acids or bases, a high activated intensity and a high coloration rate.

### DETAILED DESCRIPTION OF THE INVENTION

In recent years, photochromic plastic materials, particularly plastic materials for optical applications, have been the subject of considerable attention. In particular, photochromic ophthalmic plastic lenses have been investigated because of the weight advantage they offer, vis-à-vis, glass lenses. Moreover, photochromic transparencies for vehicles, such as cars and airplanes, have been of interest because of the potential safety features that such transparencies offer.

In accordance with the present invention, it has now been discovered that certain novel dioxino, oxazino, pyrimidino and pyrano-fused naphtho[1,2-b]pyrans having activated colors ranging from red to violet, an acceptable fade rate, high activated intensity and a high coloration rate may be prepared. These compounds may be described as naphtho[1,2-b]pyrans having a six-membered heterocyclic ring fused to the f side of the basic naphthopyran structure and having certain substituents at the position ortho to the oxygen atom of the basic naphthopyran. In particular, the compounds include dioxino[5',4':3,4]naphtho[1,2-b]pyrans, oxazino[5',4':3,4]naphtho[1,2-b]pyrans, oxazino[5',6':3,4]naphtho[1,2-b]pyrans, pyrano[3',4':3,4]naphtho[1,2-b]pyrans and pyrimidino[5',4':3,4]naphtha[1,2-b]pyrans, each having an oxo group substituted at the number 4 position and certain substituents at the number 2 position with certain other substituents optionally present at the number 9, 10, 11 or 12 positions.

These aforedescribed compounds may be represented by the following graphic formula I in which the letters a through n represent the sides of the naphthopyran, and the numbers 1 through 12 inside the rings identify the numbering sequence of the ring atoms of the six-membered heterocyclic-fused naphthopyran.

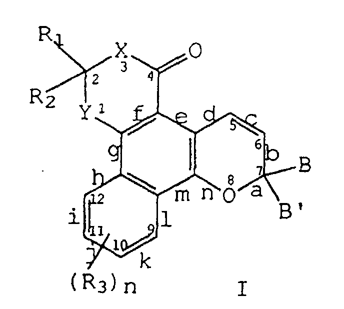

In graphic formula I, R₁ and R₂ may together form an oxo group. Alternatively, R₁ is hydrogen and R₂ may be hydrogen, C₁-C₆ alkyl, C₃-C₇ cycloalkyl, allyl, phenyl, mono-and di-substituted phenyl, benzyl, mono-substituted benzyl, naphthyl, mono- and di-substituted naphthyl, C₄-C₁₂ bicycloalkyl, linear or branched C₃-C₁₂ alkenyl, C₁-C₆ alkoxycarbonyl(C₁-C₆)alkyl, methacryloxy(C₁-C₆)alkyl, acryloxy(C₁-C₆)alkyl, C₁-C₄ acyloxy(C₁-C₆)alkyl or C₁-C₆ alkoxy(C₁-C₆)alkyl or the unsubstituted, mono- or di-substituted heteroaromatic groups pyridyl, furanyl, benzofuran-2-yl, benzyfuran-3-yl, thienyl, benzothien-2-yl, benzothien-3-yl, dibenzofuranyl, dibenzothienyl, carbazolyl, benzopyridyl and indolyl. Each of the phenyl, benzyl, naphthyl and heteroaromatic group substituents may be C₁-C₆ alkyl, C₁-C₆ alkoxy, morpholino, di(C₁-C₆)alkylamino, chloro or fluoro. Preferably, R₂ is selected from the group consisting of hydrogen, C₁-C₅ alkyl, C₃-C₆ cycloalkyl, phenyl, mono- or di-substituted phenyl, benzyl and mono-substituted benzyl. Each of the preferred phenyl and benzyl group substituents are C₁-C₄ alkyl or C₁-C₄ alkoxy. More preferably, R₂ is selected from the group consisting of hydrogen, C₁-C₅ alkyl and phenyl.

Each R₃ in graphic formula I is selected from the group consisting of C₁-C₆ alkyl, C₁-C₆ alkoxy, chloro, fluoro, phenyl, mono- and di-substituted phenyl, benzyl or mono-substituted benzyl, C₃-C₇ cycloalkyl, aryloxy, di(C₁-C₆)alkylamino, morpholino, thiomorpholino, piperidino, pyridyl, tetrahydroquinolino, isoquinolino, aziridino, diarylamino, N-(C₁-C₆)alkyl piperizino and N-aryl piperizino, wherein the aryl groups are phenyl or naphthyl and n is the integer 0, 1 or 2. The phenyl and benzyl substituents are C₁-C₆ alkyl, C₁-C₆ alkoxy, fluoro or chloro. Preferably, each R₃ is selected from the group consisting of C₁-C₄ alkyl, C₁-C₄ alkoxy, fluoro, phenyl and aryloxy, and n is the integer 0, 1, or 2. More preferably, each R₃ is selected from the group consisting of C₁-C₃ alkyl, C₁-C₃ alkoxy, phenyl and aryloxy and n is the integer 0, 1 or 2.

X in graphic formula I may be oxygen or -N(R₄)-, wherein R₄ is hydrogen, C₁-C₆ alkyl, C₃-C₇ cycloalkyl, allyl, vinyl, C₁-C₅ acyl, phenyl, mono- and di-substituted phenyl, benzyl, mono-substituted benzyl, C₁-C₄ alkoxycarbonyl(C₁-C₆)alkyl, methacryloxy(C₁-C₆)alkyl, acryloyloxy(C₁-C₆)alkyl, phenyl(C₁-C₆)alkyl, naphthyl, C₄-C₁₂ bicycloalkyl or C₂-C₄ acyloxy or the unsubstituted or substituted heteroaromatic groups pyridyl, furanyl, benzofuran-2-yl, benzofuran-3-yl, thienyl, benzothien-2-yl, benzothien-3-yl, dibenzofuranyl, dibenzothienyl, carbazolyl, benzopyridyl and indolyl. Each of the phenyl, benzyl and heteroaromatic group substituents may be C₁-C₆ alkyl or C₁-C₆ alkoxy. Preferably, X is oxygen or -NR₄-, wherein R₄ is hydrogen, C₁-C₃ alkyl, methacryloxy(C₁-C₆)alkyl or acryloxy(C₁-C₆)alkyl.

Y in graphic formula I may be oxygen, -N(R₄)- or -C((R₅)R₆)-, with the proviso that when Y is C((R₅)R₆)-, X is oxygen. R₄ is the same as described for X, R₅ and R₆ may each be hydrogen, C₁-C₆ alkyl or C₃-C₇ cycloalkyl. Preferably, Y is oxygen, -NH- or -CH₂-.

B and B' in graphic formula I may each be selected from the group consisting of:
(i) the unsubstituted, mono-, di- and tri-substituted aryl groups, phenyl and naphthyl;
(ii) the unsubstituted, mono- and di-substituted heteroaromatic groups pyridyl, furanyl, benzofuran-2-yl, benzofuran-3-yl, thienyl, benzothien-2-yl, benzothien-3-yl, dibenzofuranyl, dibenzothienyl, carbazolyl benzopyridyl, indolyl and fluorenyl, each of said aryl and heteroaromatic substituents in parts (i) and (ii) being selected from the group consisting of hydroxy, aryl, i.e., phenyl and naphthyl, mono(C₁-C₆)alkoxyaryl, di(C₁-C₆)alkoxyaryl, mono(C₁-C₆)alkylaryl, di(C₁-C₆)alkylaryl, chloroaryl, fluoroaryl, C₃-C₇ cycloalkylaryl, C₃-C₇ cycloalkyl, C₃-C₇ cycloalkyloxy, C₃-C₇ cycloalkyloxy(C₁-C₆)alkyl, C₃-C₇ cycloalkyloxy(C₁-C₆)alkoxy, aryl(C₁-C₆)alkyl, aryl(C₁-C₆)alkoxy, aryloxy, aryloxy(C₁-C₆)alkyl, aryloxy(C₁-C₆)alkoxy, mono- and di-(C₁-C₆)alkylaryl(C₁-C₆)alkyl, mono- and di-(C₁-C₆)alkoxyaryl(C₁-C₆)alkyl, mono- and di-(C₁-C₆)alkylaryl(C₁-C₆)alkoxy, mono- and di-(C₁-C₆)alkoxyaryl(C₁-C₆)alkoxyl amino, mono(C₁-C₆)alkylamino, di(C₁-C₆)alkylamino, diarylamino, piperazino, N-(C₁-C₆)alkylpiperazino, N-arylpiperazino, aziridino, indolino, piperidino, morpholino, thiomorpholino, tetrahydroquinolino, tetrahydroisoquinolino, pyrryl, C₁-C₆ alkyl, C₁-C₆ chloroalkyl, C₁-C₆ fluoroalkyl, C₁-C₆ alkoxy, mono(C₁-C₆)alkoxy(C₁-C₄)alkyl, acryloxy, methacryloxy, bromo, chloro and fluoro;
(iii) the groups represented by the following graphic formulae: 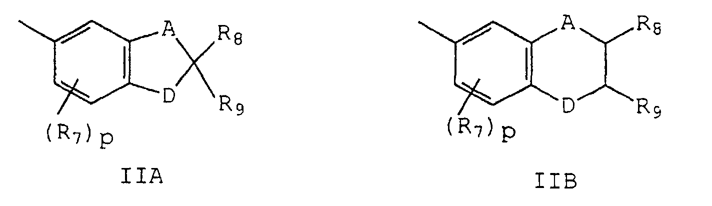 wherein A may be carbon or oxygen and D may be oxygen or substituted nitrogen, provided that when D is substituted nitrogen, A is carbon, said nitrogen substituents being selected from the group consisting of hydrogen, C₁-C₆ alkyl and C₂-C₆ acyl; each R₇ is C₁-C₆ alkyl, C₁-C₆ alkoxy, hydroxy, chloro or fluoro; R₈ and R₉ are each hydrogen or C₁-C₆ alkyl; and p is the integer 0, 1 or 2;
(iv) C₁-C₆ alkyl, C₁-C₆ chloroalkyl, C₁-C₆ fluoroalkyl, C₁-C₆ alkoxy(C₁-C₄)alkyl, C₃-C₆ cycloalkyl, mono(C₁-C₆)alkoxy(C₃-C₆)cycloalkyl, mono(C₁-C₆)alkyl(C₃-C₆)cycloalkyl, chloro(C₃-C₆)cycloalkyl, fluoro(C₃-C₆)cycloalkyl and C₄-C₁₂ bicycloalkyl; and
(v) the group represented by the following graphic formula: 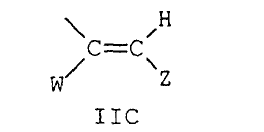 wherein W in graphic formula IIC may be hydrogen or C₁-C₄ alkyl and Z in graphic formula IIC may be selected from the unsubstituted, mono-, and di-substituted members of the group consisting of naphthyl, phenyl, furanyl and thienyl, each of said group substituents in this part (v) being C₁-C₄ alkyl, C₁-C₄ alkoxy, fluoro or chloro; or
(vi) B and B' taken together form fluoren-9-ylidene, mono-, or di-substituted fluoren-9-ylidene or form a member selected from the group consisting of saturated C₃-C₁₂ spiro-monocyclic hydrocarbon rings, e.g., cyclopropylidene, cyclobutylidene, cyclopentylidene, cyclohexylidene, cycloheptylidene, cyclooctylidene, cyclononylidene, cyclodecylidene, cycloundecylidene, cyclododecylidene; saturated C₇-C₁₂ spirobicylic hydrocarbon rings, e.g., bicyclo[2.2.1]heptylidene, i.e., norbornylidene, 1,7,7-trimethyl bicyclo[2.2.1]heptylidene, i.e., bornylidene, bicyclo[3.2.1]octylidene, bicyclo[3.3.1]nonan-9-ylidene, bicyclo[4.3.2]undecane, and saturated C₇-C₁₂ spiro-tricyclic hydrocarbon rings, e.g., tricyclo[2.2.1.0^{2,6}]heptylidene, tricyclo[3.3.1.1^{3,7}]decylidene, i.e., adamantylidene, and tricyclo[5.3.1.1^{2,6}]dodecylidene, each of said fluoren-9-ylidene substituents being selected from the group consisting of C₁-C₄ alkyl, C₁-C₄ alkoxy, fluoro and chloro.

More preferably, B and B' are each selected from the group consisting of:
(i) phenyl, mono-substituted phenyl and di-substituted phenyl;
(ii) the unsubstituted, mono- and di-substituted heteroaromatic groups furanyl, benzofuran-2-yl, thienyl, benzothien-2-yl, dibenzofuranyl, aryloxy and diarylamino, each of said phenyl and heteroaromatic substituents in parts (i) and (ii) being selected from the group consisting of di(C₁-C₃)alkylamino, piperidino, morpholino, pyrryl, C₁-C₃ alkyl, C₁-C₃ chloroalkyl, C₁-C₃ fluoroalkyl, C₁-C₃ alkoxy, mono(C₁-C₃)alkoxy(C₁-C₃)alkyl, fluoro and chloro;
(iii) the groups represented by the graphic formulae IIA and IIB, wherein A is carbon and D is oxygen, R₇ is C₁-C₃ alkyl or C₁-C₃ alkoxy, R₈ and R₉ are each hydrogen or C₁-C₄ alkyl, and p is the integer 0 or 1;
(iv) C₁-C₄ alkyl; and
(v) the group represented by the graphic formula IIC wherein W is hydrogen or methyl and Z is phenyl or mono-substituted phenyl, said phenyl substituent being selected from the group consisting of C₁-C₃ alkyl, C₁-C₃ alkoxy and fluoro; or
(vi) B and B' taken together form fluoren-9-ylidene, mono-substituted fluoren-9-ylidene or a member selected from the group consisting of saturated C₃-C₈ spiro-monocyclic hydrocarbon rings, saturated C₇-C₁₀ spiro-bicyclic hydrocarbon rings, and saturated C₇-C₁₀ spiro-tricyclic hydrocarbon rings, said fluoren-9-ylidene substituent being selected from the group consisting of C₁-C₃ alkyl, C₁-C₃ alkoxy, fluoro and chloro.

Most preferably, B and B' are each selected from the group consisting of:
(i) phenyl, mono- and di-substituted phenyl;
(ii) the unsubstituted, mono- and di-substituted heteroaromatic groups furanyl, benzofuran-2-yl, thienyl, benzothien-2-yl, dibenzofuranyl, aryloxy and diarylamino, each of said phenyl and heteroaromatic substituents in parts (i) and (ii) being selected from the group consisting of C₁-C₃ alkyl, C₁-C₃ alkoxy and fluoro; and
(iii) the group represented by graphic formula IIA, wherein A is carbon and D is oxygen, R₇ is C₁-C₃ alkyl or C₁-C₃ alkoxy, R₈ and R₉ are each hydrogen or C₁-C₃ alkyl, and p is the integer 0 or 1; or
(iv) B and B' taken together form fluoren-9-ylidene, adamantylidene, bornylidene, norbornylidene, or bicyclo[3.3.1]nonan-9-ylidene.

The compounds represented by graphic formula I wherein X and Y are each oxygen and are prepared according to Reactions A-D. Benzophenones represented by graphic formula V and VA are either purchased or prepared by Friedel-Crafts methods using an appropriately substituted or unsubstituted benzoyl chloride of graphic formula IV and a commercially available substituted or unsubstituted benzene compound of graphic formula III. See the publication Friedel-Crafts and Related Reactions, George A. Olah, Interscience Publishers, 1964, Vol. 3, Chapter XXXI (Aromatic Ketone Synthesis), and "Regioselective Friedel-Crafts Acylation of 1,2,3,4-Tetrahydroquinoline and Related Nitrogen Heterocycles: Effect on NH Protective Groups and Ring Size" by Ishihara, Yugi et al, J. Chem. Soc., Perkin Trans. 1, pages 3401 to 3406, 1992.

The compounds represented by graphic formulae III and IV are dissolved in a solvent, such as carbon disulfide or methylene chloride, and reacted in the presence of a Lewis acid, such as aluminum chloride or tin tetrachloride, to form the corresponding substituted benzophenone represented by graphic formula V (or VA in Reaction B). R and R' represent potential phenyl substituents, as described hereinbefore.

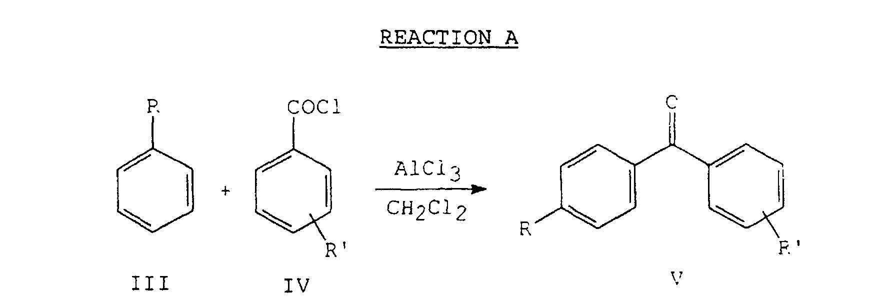

In Reaction B, the substituted or unsubstituted ketone represented by graphic formula VA, in which B and B' may represent groups other than substituted or unsubstituted phenyl, is reacted with sodium acetylide in a suitable solvent, such as anhydrous tetrahydrofuran (THF), to form the corresponding propargyl alcohol represented by graphic formula VI. Propargyl alcohols having B or B' groups other than substituted and unsubstituted phenyl may be prepared from commercially available ketones or for example, from ketones prepared via reaction of an acyl halide with a substituted or unsubstituted benzene, naphthalene, or heteroaromatic compound. Propargyl alcohols having B or B' groups represented by graphic formula IIC may be prepared by the methods described in U.S. Patent 5,274,132, column 2, lines 40 to 68.

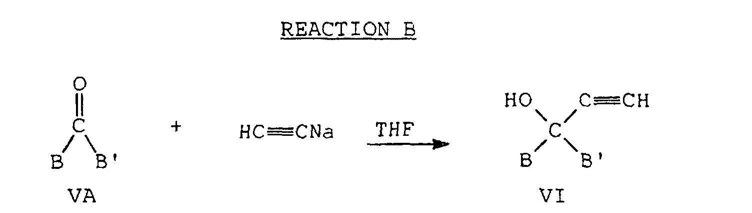

In Reaction C, the naphthol represented by graphic formula VII is prepared by the methods disclosed in U.S. Patent 5,162,570, incorporated herein by reference, and coupled with the propargyl alcohol represented by graphic formula VI in the presence of a catalytic amount of an acid, e.g., p-toluene sulfonic acid in a suitable solvent, such as toluene or chlcroform, to produce the 5-phenoxycarbonyl-6-hydroxy naphtho[1,2-b]pyran represented by graphic formula VIII.

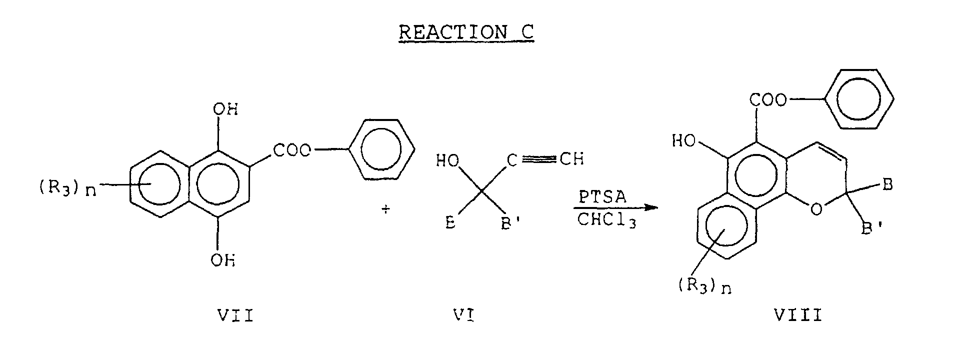

In Reaction D, the naphthopyran represented by graphic formula VIII is reacted with a commercially available aldehyde (R"CHO) in the presence of a slight excess of an amine, e.g., triethylamine (NEt₃) or DABCO, i.e., 1,4-diazabicyclo(2.2.2)octane or triethylenediamine, in a suitable solvent such as chloroform, to form compounds represented by graphic formula IX. R" represents the individual substituents that R₂ may be and R₁ is hydrogen.

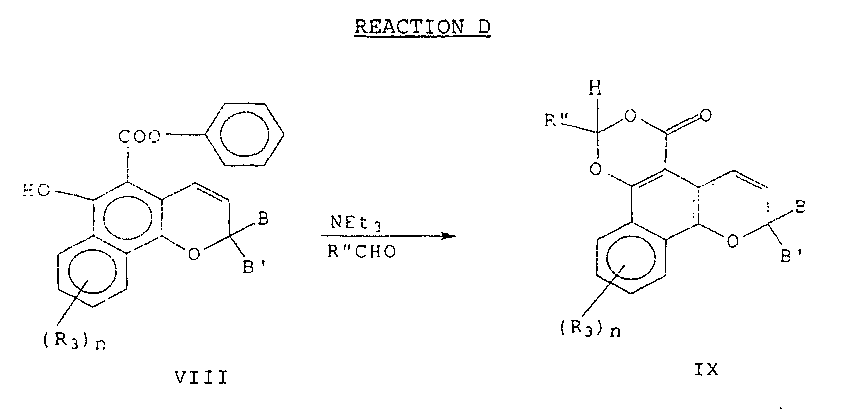

The compounds represented by graphic formula I wherein X is -N(R₄)- and Y is oxygen are prepared according to Reaction E as follows. The naphthopyran represented by graphic formula X, prepared according to the methods disclosed in U.S. Patent 5,458,814 (hereinafter the '814 patent), is reacted with an isocyanate (R₄NCO) in the presence of triethylamine and a catalytic amount of 4-dimethylaminopyridine (DMAP) in a suitable solvent, such as dimethyl formamide (DMF) to form the [1,3]oxazino[5',6':3,4]naphtho[1,2-b]pyran represented by graphic formula XI.

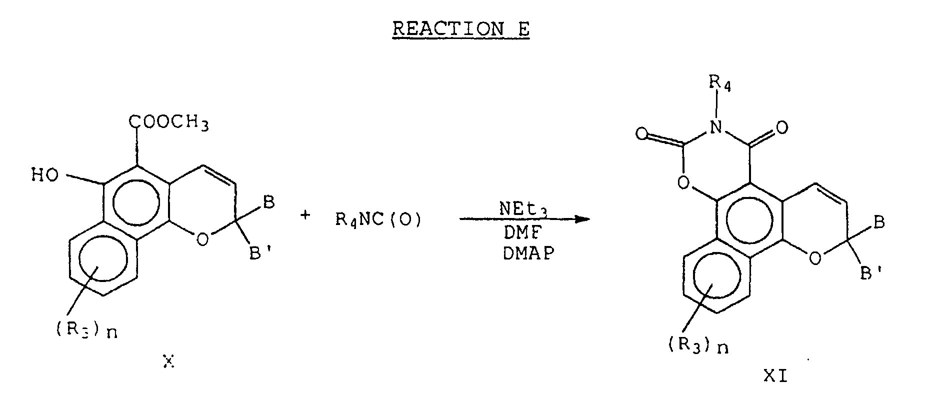

Alternatively, the compounds represented by graphic formula I wherein X is oxygen or -N(R₄)- and Y is -N(R₄)-, e.g., where R₄ is hydrogen, may be prepared according to Reaction F. The procedure of Reaction F may also be used to prepare compounds of graphic formula I when R₄ is a substituent other than hydrogen. The naphthopyran represented by graphic formula XA, which may be prepared according to the '814 patent, is reacted with the substituted imino Grignard reagent represented by graphic formula XII in a suitable solvent, such as THF, to produce the corresponding pyrimido-fused naphthopyran represented by graphic formula XIII as the major product and the oxazino-fused naphthopyran represented by graphic formula XIV as the minor product.

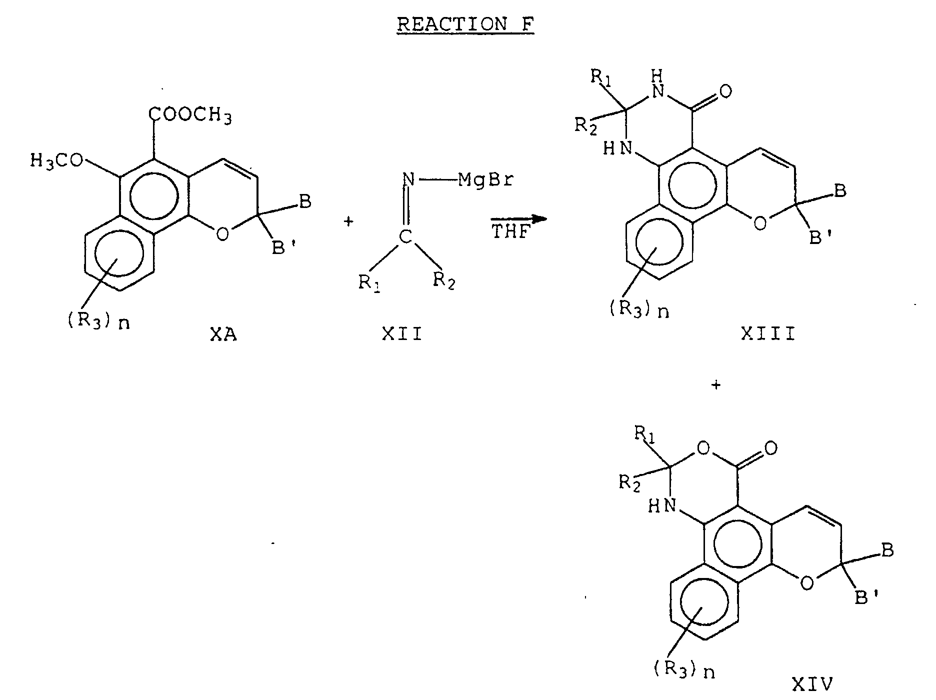

The compounds represented by graphic formula I wherein X is oxygen and Y is -C((R₅)R₆)-, e.g., -CH₂-, are prepared according to Reactions G and H. In Reaction G, the naphthopyran represented by graphic formula XA is reacted with the vinylic Grignard reagent represented by graphic formula XV in a suitable solvent, such as THF, yielding the corresponding substituted naphthopyran represented by graphic formula XVI.

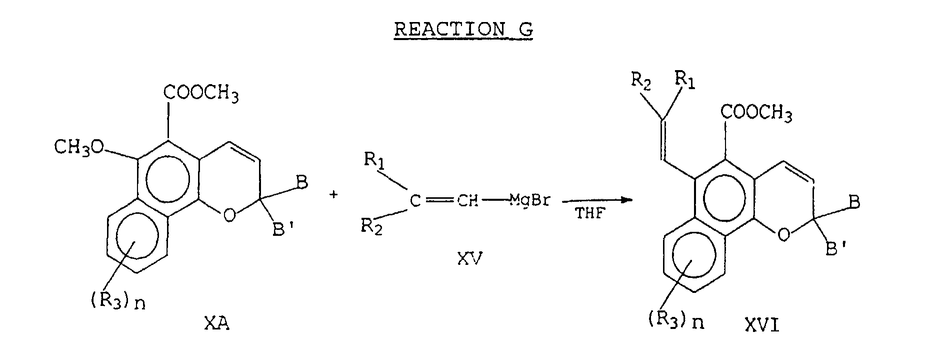

In Reaction H, compound XVI is cyclized by reaction with trimethyl silyl chloride in the presence of sodium iodide in a suitable solvent, such as acetonitrile (ACN), to form compounds represented by graphic formula XVII.

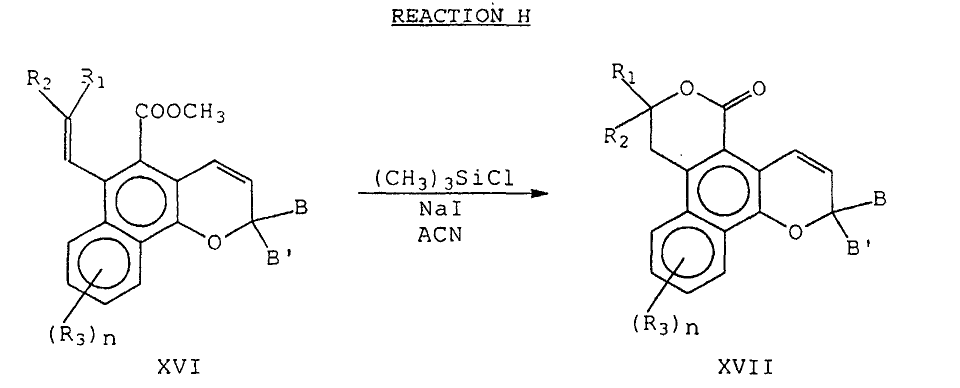

Compounds represented by graphic formula I may be used in those applications in which organic photochromic substances may be employed, such as optical lenses, e.g., vision correcting ophthalmic lenses and piano lenses, face shields, goggles, visors, camera lenses, windows, automotive windshields, aircraft and automotive transparencies, e.g., T-roofs, sidelights and backlights, plastic films and sheets, textiles and coatings, e.g., coating compositions such as paints, and verification marks on security documents, e.g., documents such as banknotes, passports and drivers' licenses for which authentication or verification of authenticity may be desired. The six membered heterocyclic-fused naphthopyrans represented by graphic formula I exhibit color changes from colorless to colors ranging from red to violet.

Examples of contemplated naphthopyran compounds within the scope of the invention include the following:
a) 7,7-diphenyl-4-oxo-4H-7H-[1,3]dioxino[5',4':3,4]naphtho[1,2-b]pyran;
b) 7,7-di(4-methoxyphenyl)-4-oxo-4H-7H-[1,3]dioxino[5',4':3,4]naphtho[1,2-b]pyran;
c) 7- (4-methoxyphenyl)-7-phenyl-2-pentyl-4-oxo-4H-7H-[1,3]dioxino[5',4':3,4]naphtho[1,2-b]pyran;
d) 7,7-diphenyl-2-ethyl-4-oxo-4H-7H-[1,3]dioxino[5',4':3,4]naphtho[1,2-b]pyran;
e) 7,7-diphenyl-2-pentyl-4-oxo-4H-7H-[1,3]dioxino[5',4':3,4]naphtho[1,2-b]pyran;
f) 7,7-diphenyl-2-(2-methylpropyl)-4-oxo-4H-7H-[1,3]dioxino[5',4':3,4]naphtho[1,2-b]pyran;
g) 2,7,7-triphenyl-4-oxo-4H-7H-[1,3]dioxino[5',4':3,4]naphtho[1,2-b]pyran;
h) 7,7-diphenyl-2-(1-phenylethyl)-4-oxo-4H-7H-[1,3]dioxino[5',4':3,4]naphtho[1,2-b]pyran;
i) 3-methyl-7,7-diphenyl-2,4-dioxo-2,3,4,7-tetrahydro[1,3]oxazino[5',6':3,4]naphtho[1,2-b]pyran;
j) 3-(2-ethoxycarbonylethyl)-7,7-diphenyl-2,4-dioxo-2,3,4,7-tetrahydro[1,3]oxazino[5',6':3,4]naphtho[1,2-b]pyran;
k) 3-hexyl-7,7-diphenyl-2,4-dioxo-2,3,4,7-tetrahydro[1,3]oxazino[5',6':3,4]naphtho[1,2-b]pyran;
l) 3-(2-methacryloyloxyethyl)-7,7-diphenyl-2,4-dioxo-2,3,4,7-tetrahydro[1,3]oxazino[5',6':3,4]naphtho[1,2-b]pyran;
m) 2,2,7,7-tetraphenyl-4-oxo-2,3,4,7-tetrahydro-1H-pyrimidino[5',4':3,4]naphtho[1,2-b]pyran;
n) 2,2,7,7-tetraphenyl-4-oxo-2,3,4,7-tetrahydro-1H-[1,3]oxazino[5',4':3,4]naphtho[1,2-b]pyran; and
o) 7,7-diphenyl-1,2,4,7-tetrahydro-2,2-dimethylpyrano[3',4':3,4]naphtho[1,2-b]pyran.

It is contemplated that the organic photochromic naphthopyrans of the present invention may be used alone, in combination with other naphthopyrans of the present invention, or in combination with one or more other appropriate complementary organic photochromic materials, i.e., organic photochromic compounds having at least one activated absorption maxima within the range of between about 400 and 700 nanometers, or substances containing same, and may be incorporated, e.g., dissolved or dispersed, in a polymeric organic host material used to prepare photochromic articles and which color when activated to an appropriate hue.

Other than where otherwise indicated, all numbers expressing wavelengths, quantities of ingredients or reaction conditions used herein are to be understood as modified in all instances by the term "about".

Examples of complementary organic photochromic compounds include other naphthopyrans, chromenes and oxazines, substituted 2H-phenanthro[4,3-b]pyran and 3H-phenanthro[1,2-b]pyran compounds, benzopyran compounds having substituents at the 2-position of the pyran ring including a dibenzo-fused 5 member heterocyclic compound and a substituted or unsubstituted heterocyclic ring, such as a benzothieno or benzofurano ring fused to the benzene portion of the benzopyrans, spiro(benzindoline)naphthopyrans, spiro(indoline)benzopyrans, spiro(indoline)naphthopyrans, spiro(indoline)quinopyrans, spiro(indoline)pyrans, spiro(indoline)naphthoxazines, spiro(indoline)pyridobenzoxazines, spiro(benzindoline)pyridobenzoxazines, spiro(benzindoline)naphthoxazines, spiro(indoline)benzoxazines, and mixtures of such photochromic compounds. Many of such photochromic compounds are described in the open literature, e.g., U.S. Patents 3,562,172; 3,567,605; 3,578,602; 4,215,010; 4,342,668; 4,816,584; 4,818,096; 4,826,977; 4,880,667; 4,931,219; 5,066,818; 5,238,931; 5,274,132; 5,384,077; 5,405,958; 5,429,774; 5,458,814; 5,466,398; 5,514,817; 5,552,090; 5,552,091; 5,565,147; 5,573,712; 5,578,252; 5,637,262; 5,645,767; 5,656,206; 5,658,500; 5,658,501; 5,674,432 and 5,698,141. Spiro(indoline)pyrans are also described in the text, Techniques in Chemistry, Volume III, "Photochromism", Chapter 3, Glenn H. Brown, Editor, John Wiley and Sons, Inc., New York, 1971.

Other complementary photochromic substances contemplated are photochromic metal-dithizonates, e.g. mercury dithizonates which are described in, for example, U.S. Patent 3,361,706, fulgides and fulgimides, e.g. the 3-furyl and 3-thienyl fulgides and fulgimides which are described in U.S. Patent 4,931,220 at column 20, line 5 through column 21, line 38.

The disclosures relating to such photochromic compounds in the aforedescribed patents are incorporated herein, *in toto*, by reference. The photochromic articles of the present invention may contain one photochromic compound or a mixture of photochromic compounds, as desired.

Each of the photochromic substances described herein may be used in amounts (or in a ratio) such that an organic host material to which the photochromic compounds or mixture of compounds is applied or in which they are incorporated exhibits a desired resultant color, e.g., a substantially neutral color when activated with unfiltered sunlight, i.e., as near a neutral color as possible given the colors of the activated photochromic compounds. Neutral gray and neutral brown colors are preferred.

A neutral gray color exhibits a spectrum that has relatively equal absorption in the visible range between 400 and 700 nanometers. A neutral brown color exhibits a spectrum in which the absorption in the 400-550 nanometer range is moderately larger than in the 550-700 nanometer range. An alternative way of describing color is in terms of its chromaticity coordinates, which describe the qualities of a color in addition to its luminance factor, i.e., its chromaticity. In the CIE system, the chromaticity coordinates are obtained by taking the ratios of the tristimulus values to their sum, e.g., x=X/(X+Y+Z) and y=Y/(X+Y+Z). Color as described in the CIE system can be plotted on a chromaticity diagram, usually a plot of the chromaticity coordinates x and y. See pages 47-52 of Principles of Color Technology, by F. W. Billmeyer, Jr., and Max Saltzman, Second Edition, John Wiley and Sons, N.Y. (1981). As used herein, a near neutral color is one in which the chromaticity coordinate values of "x" and "y" for the color are within the following ranges (D65 illuminant): x = 0.260 to 0.400, y = 0.280 to 0.400 following activation to 40 percent luminous transmission by exposure to solar radiation (Air Mass 1 or 2).

The amount of photochromic substance or composition containing same applied to or incorporated into a host material is not critical provided that a sufficient amount is used to produce a photochromic effect discernible to the naked eye upon activation. Generally such amount can be described as a photochromic amount. The particular amount used depends often upon the intensity of color desired upon irradiation thereof and upon the method used to incorporate or apply the photochromic substances. Typically, the more photochromic substance applied or incorporated, the greater is the color intensity up to a certain limit.

The relative amounts of the aforesaid photochromic compounds used will vary and depend in part upon the relative intensities of the color of the activated species of such compounds, and the ultimate color desired. Generally, the amount of total photochromic substance incorporated into or applied to a photochromic optical host material may range from 0.05 to 1.0, e.g., from 0.1 to 0.45, milligrams per square centimeter of surface to which the photochromic substance(s) is incorporated or applied.

The photochromic substances of the present invention may be applied to or incorporated into a host material such as a polymeric organic host material by various methods described in the art. Such methods include dissolving or dispersing the photochromic substance within the host material, e.g., casting it in place by adding the photochromic substance to the monomeric host material prior to polymerization; imbibition of the photochromic substance into the host material by immersion of the host material in a hot solution of the photochromic substance or by thermal transfer; providing the photochromic substance as a separate layer between adjacent layers of the host material, e.g., as a part of a polymeric film; applying the photochromic substance as part of a coating or. film placed on the surface of the host material; and applying a photochromic polymeric overlay section to the surface of the host material. The overlay section may have a vision correcting feature. The term "imbibition" or "imbibe" is intended to mean and include permeation of the photochromic substance alone into the host material, solvent assisted transfer of the photochromic substance into a porous polymer, vapor phase transfer, and other such transfer mechanisms.

Compatible (chemically and color-wise) tints, i.e., dyes, may be applied to the host material to achieve a more aesthetic result, for medical reasons, or for reasons of fashion. The particular dye selected will vary and depend on the aforesaid need and result to be achieved. In one embodiment, the dye may be selected to complement the color resulting from the activated photochromic substances, e.g., to achieve a more neutral color or absorb a particular wavelength of incident light. In another embodiment, the dye may be selected to provide a desired hue to the host matrix when the photochromic substances is in an unactivated state.

The host material will usually be transparent, but may be translucent or even opaque. The host material need only be transparent to that portion of the electromagnetic spectrum, which activates the photochromic substance, i.e., that wavelength of ultraviolet (UV) light that produces the open form of the substance and that portion of the visible spectrum that includes the absorption maximum wavelength of the substance in its UV activated form, i.e., the open form. Preferably, the host color should not be such that it masks the color of the activated form of the photochromic substance, i.e., so the change in color is readily apparent to the observer. More preferably, the host material article is a solid transparent or optically clear material, e.g., materials suitable for optical applications, such as plano and ophthalmic lenses, windows, automotive transparencies, e.g., windshields, aircraft transparencies, plastic sheeting, polymeric films, etc.

The photochromic compounds of the present invention may be present in an organic solvent or an organic polymeric host. The organic solvent may be selected from the group consisting of benzene, toluene, methyl ethyl ketone, acetone, ethanol, tetrahydrofurfuryl alcohol, N-methyl pyrrolidinone, 2-methoxyethyl ether, xylene, cyclohexane, 3-methyl cyclohexanone, ethyl acetate, tetrahydrofuran, methanol, methyl propinate, ethylene glycol and mixtures thereof. Preferably, the organic solvent is selected from the group consisting of acetone, ethanol, tetrahydrofurfuryl alcohol, 2-methoxyethyl ether, 3-methyl cyclohexanone, N-methyl pyrrolidinone and mixtures thereof.

Preferably, the organic polymeric host material is a solid transparent or optically clear material, e.g., materials suitable for optical applications, such as plano and ophthalmic lenses, windows, automotive transparencies, e.g., windshields, aircraft transparencies, plastic sheeting, polymeric films, etc.

Examples of polymeric organic host materials are polymers prepared from individual monomers or mixtures of monomers selected from the following groups:
(a) diacrylate or dimethacrylate compounds represented by graphic formula XVIII: 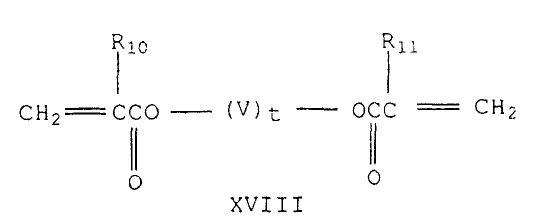 wherein R₁₀ and R₁₁ may be the same or different and are hydrogen or methyl, V is (CH₂), and t is an integer of from 1 to 20;
(b) diacrylate or dimethacrylate compounds represented by graphic formula XIX: 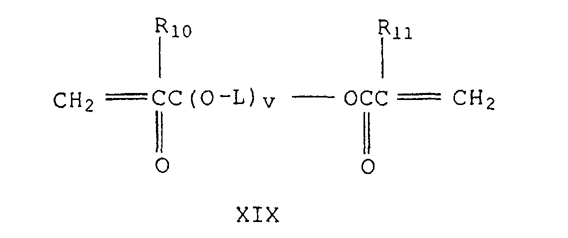 wherein L is a straight or branched chain alkylene containing from 2 to 4 carbon atoms, and v is an integer of from 1 to 50; and
(c) an acrylate or a methacrylate compound having an epoxy group represented by graphic formula XX:

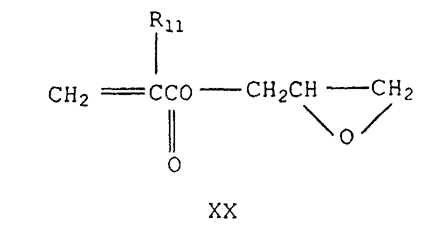

In graphic formulae XVIII, XIX and XX, like letters used with respect to the definitions of different substituents have the same meaning.

Examples of diacrylate or dimethacrylate compounds, i.e., di(meth)acrylates, represented by graphic formula XVIII include butanediol di(meth)acrylate, hexanediol di(meth)acrylate and nonanediol di(meth)acrylate, and represented by graphic formula XIX include diethylene glycol dimethacrylate, triethylene glycol dimethacrylate and poly(oxyalkylene dimethacrylates), e.g., polyethylene glycol (600) dimethacrylate. Examples of acrylate or methacrylate compounds represented by graphic formula XX include glycidyl acrylate and glycidyl methacrylate.

Further examples of polymeric organic host materials which may be used with the photochromic compounds described herein include: polymers, i.e., homopolymers and copolymers, of the monomers and mixtures of monomers represented by graphic formulae XVIII, XIX and XX, bis(allyl carbonate) monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers, alkoxylated polyhydric alcohol polyacrylate monomers, such as ethoxylated trimethylol propane triacrylate monomers, urethane acrylate monomers, such as those described in U.S. Patent 5,373,033, and vinylbenzene monomers, such as those described in U.S. Patent 5,475,074 and styrene; polymers, i.e., homopolymers and copolymers, of polyfunctional, e.g., mono-, di- or multi-functional, acrylate and/or methacrylate monomers, poly(C₁-C₁₂ alkyl methacrylates), such as poly(methyl methacrylate), poly(alkoxylated phenol methacrylates), cellulose acetate, cellulose triacetate, cellulose acetate propionate, cellulose acetate butyrate, poly(vinyl acetate), poly(vinyl alcohol), poly(vinyl chloride), poly(vinylidene chloride), polyurethanes, polythiourethanes, thermoplastic polycarbonates, polyesters, poly(ethylene terephthalate), polystyrene, poly(alpha methylstyrene), copoly(styrene-methyl methacrylate), copoly(styrene-acrylonitrile), polyvinylbutyral and polymers, i.e., homopolymers and copolymers, of diallylidene pentaerythritol, particularly copolymers with polyol (allyl carbonate) monomers, e.g., diethylene glycol bis(allyl carbonate), and acrylate monomers, e.g., ethyl acrylate, butyl acrylate.

Transparent copolymers and blends of transparent polymers are also suitable as host materials. Preferably, the host material is an optically clear polymerized organic material prepared from a thermoplastic polycarbonate resin, such as the carbonate-linked resin derived from bisphenol A and phosgene, which is sold under the trademark, LEXAN; a polyester, such as the material sold under the trademark, MYLAR; a poly(methyl methacrylate), such as the material sold under the trademark, PLEXIGLAS; polymerizates of a polyol(allyl carbonate) monomer, especially diethylene glycol bis(allyl carbonate), which monomer is sold under the trademark CR-39, and polymerizates of copolymers of a polyol (allyl carbonate), e.g., diethylene glycol bis(allyl carbonate), with other copolymerizable monomeric materials, such as copolymers with vinyl acetate, e.g., copolymers of from 80-90 percent diethylene glycol bis(allyl carbonate) and 10-20 percent vinyl acetate, particularly 80-85 percent of the bis(allyl carbonate) and 15-20 percent vinyl acetate, and copolymers with a polyurethane having terminal diacrylate functionality, as described in U.S. patents 4,360,653 and 4,994,208; and copolymers with aliphatic urethanes, the terminal portion of which contain allyl or acrylyl functional groups, as described in U.S. Patent 5,200,483; poly(vinyl acetate), polyvinylbutyral, polyurethane, polythrourethane and polymers of members of the group consisting of diethylene glycol dimethacrylate monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers and ethoxylated trimethylol propane triacrylate monomers; cellulose acetate, cellulose propionate, cellulose butyrate, cellulose acetate butyrate, polystyrene and copolymers of styrene with methyl methacrylate, vinyl acetate and acrylonitrile.

More particularly, contemplated is use of the photochromic naphthopyrans of the present invention with optical organic resin monomers used to produce optically clear polymerizates, i.e., materials suitable for optical applications, such as for example plano and ophthalmic lenses, windows, and automotive transparencies. Such optically clear polymerizates may have a refractive index that may range from about 1.48 to about 1.75, e.g., from about 1.495 to about 1.66. Specifically contemplated are optical resins sold by PPG Industries, Inc. under the CR- designation, e.g., CR-307 and CR-407.

The present invention is more particularly described in the following examples which are intended as illustrative only, since numerous modifications and variations therein will be apparent to those skilled in the art.

### EXAMPLE 1

### Step 1

Phenyl-1,4-dihydroxy-2-naphthoate (5 grams) and 1,1-diphenyl-2-propyn-1-ol (4 grams) were added to a reaction flask containing 100 milliliters (mL) chloroform and stirred at room temperature. p-Toluene sulfonic acid (0.1 gram) was added to the reaction mixture and stirred for 10 hours. The solvent was evaporated leaving a residue which crystallized in diethyl ether. The crystals were separated via filtration, washed with hexane and oven dried yielding 6 grams of a product having a melting point of 158-160°C. A nuclear magnetic resonance (NMR) spectrum showed the product to have a structure consistent with 2,2-diphenyl-5-phenoxycarbonyl-6-hydroxy-2H-naphtho[1,2-b]pyran.

### Step 2

One gram of the naphthopyran produced in Step 1 and triethylamine (0.5 gram) were added to a reaction flask containing chloroform (50 mL) and mixed. Paraformaldehyde (2.5 grams) was added to the reaction mixture and stirred for five hours. The mixture was filtered and the solvent in the filtrate was evaporated leaving an oily residue. The residue was dissolved in diethyl ether and washed with a five percent solution of hydrochloric acid and rewashed with water. The resulting organic layer was separated, dried and concentrated. The concentrate was crystallized from 1:1 ether:hexane mixture. The crystals were filtered, washed and oven dried yielding 0.6 gram of a product having a melting point of 234-236°C. An NMR spectrum showed the product to have a structure consistent with 7,7-diphenyl-4-oxo-4H-7H-[1,3]dioxino[5'4':3,4]naphtho[1,2-b]pyran.

### EXAMPLE 2

The process of Example 1 was followed except that in Step 1, 1,1-bis(4-methoxyphenyl)-2-propyn-1-ol was used instead of 1,1-diphenyl-2-propyn-1-ol and in Step 2, two grams of the naphthopyran produced in Step 1 was used (instead of one gram thereof) and 4 grams of paraformaldehyde (instead of 2.5 grams thereof) was added to the reaction flask. Following the same workup procedure, 1.5 grams of a product having a melting point of 147-149°C was obtained. An NMR spectrum showed the product to have a structure consistent with 7,7-di (4-methoxyphenyl)-4-oxo-4H-7H-[1,3]dioxino[5',4':3,4]naphtho[1,2-b]pyran.

### EXAMPLE 3

The process of Example 1 was followed except that in Step 1, 1-(4-methoxyphenyl)-1-phenyl-2-propyn-1-ol was used instead of 1,1-diphenyl-2-propyn-1-ol and in Step 2, eight grams of the naphthopyran produced in Step 1 was used (instead of one gram thereof) and 15 grams of hexanal (instead of 2.5 grams thereof) was added to the reaction flask. Following the same workup procedure, 5 grams of a product having a melting point of 126-127°C was obtained. An NMR spectrum showed the product to have a structure consistent with 7-(4-methoxyphenyl)-7-phenyl-2-pentyl-4-oxo-4H-7H-[1,3]dioxino[5',4':3,4]naphtho[1,2-b]pyran.

### EXAMPLE 4

Two grams of the naphthopyran produced in Step 1 of Example 1, propanal (20 mL) and triethylamine (2 mL) were added to a reaction flask and stirred for three hours. Chloroform (50 mL) was added to the flask to dissolve the mixture. The solution was washed with a five percent solution of hydrochloric acid. The resulting organic layer was separated, washed with water, dried and concentrated. The concentrate crystallized from diethyl ether. The crystals were filtered, washed and oven dried yielding 1.7 grams of a product having a melting point of 163-164°C. An NMR spectrum showed the product to have a structure consistent with 7,7-diphenyl-2-ethyl-4-oxo-4H-7H-[1,3]dioxino[5',4':3,4]naphtho[1,2-b]pyran.

### EXAMPLE 5

The process of Example 4 was followed except that 10 grams of a naphthopyran produced according to Step 1 of Example 1 was used, hexanal (20 mL) was used instead of propanaldehyde and DABCO (10 grams) was used instead of triethylamine. The process yielded 9.0 grams of a product having a melting point of 156-158°C. An NMR spectrum showed the product to have a structure consistent with 7,7-diphenyl-2-pentyl-4-oxo-4H-7H-[1,3]dioxino[5',4':3,4]naphtho[1,2-b]pyran.

### EXAMPLE 6

The process of Example 4 was followed except that isovaleraldehyde was used instead of propanal yielding 1.6 grams of a product having a melting point of 158-160°C. An NMR spectrum showed the product to have a structure consistent with 7,7-diphenyl-2-(2-methylpropyl)-4-oxo-4H-7H-[1,3]dioxino[5',4':3,4]naphtho[1,2-b]pyran.

### EXAMPLE 7

The process of Example 4 was followed except that benzaldehyde was used instead of propanal yielding 1.2 grams of a product having a melting point of 191-193°C. An NMR spectrum showed the product to have a structure consistent with 2,7,7-triphenyl-4-oxo-4H-7H-[1,3]dioxino[5',4':3,4]naphtho[1,2-b]pyran.

### EXAMPLE 8

The process of Example 4 was followed except that 2-phenylpropionaldehyde was used instead of propanal yielding 1.6 grams of a product having a melting point of 206-208°C. An NMR spectrum showed the product to have a structure consistent with 7,7-diphenyl-2-(1-phenylethyl)-4-oxo-4H-7H-[1,3]dioxino[5',4':3,4]naphtho[1,2-b]pyran.

### EXAMPLE 9

2,2-Diphenyl-5-methoxycarbonyl-6-hydroxy-2H-naphtho[1,2-b]pyran (3 grams) and DABCO (3 grams) were added to a reaction flask containing anhydrous dimethylformamide (40 mL) and stirred at room temperature. Methyl isocyanate (3 mL) was added dropwise to the mixture and stirred for five hours. Water (100 mL) was added to the mixture and extracted with diethyl ether. The ether layer was separated, washed with a five percent solution of hydrochloric acid, dried and concentrated. The concentrate was purified using a silica column with chloroform as the eluant yielding 1 gram of the desired product having a melting point of 245-247°C and 1.2 grams of a byproduct having a melting point of 213-215°C. An NMR spectrum showed the desired product to have a structure consistent with 3-methyl-7,7-diphenyl-2,4-dioxo-2,3,4,7-tetrahydro[1,3]oxazino[5',6':3,4]naphtho[1,2-b]pyran and the byproduct to have a structure consistent with 2,2-diphenyl-5-methoxycarbonyl-6-(N-methylamino)carbonyloxy-2H-naphtho[1,2-b]pyran.

### EXAMPLE 10

The process of Example 9 was followed except that 5 grams of the naphthopyran were used and ethyl-1-isocyanopropionate (10 mL) was used instead of methyl isocyanate and triethylamine was used instead of DABCO. A single product was isolated and had a melting point of 187-188°C. An NMR spectrum showed the product to have a structure consistent with 3-(2-ethoxycarbonylethyl)-7,7-diphenyl-2,4-dioxo-2,3,4,7-tetrahydro[1,3]oxazino[5',6':3,4]naphtho[1,2-b]pyran.

### EXAMPLE 11

The process of Example 9 was followed except that 15 grams of the naphthopyran were used and n-hexyl isocyanate (20 mL) was used instead of methyl isocyanate and triethylamine was used instead of DABCO. A single product was isolated and had a melting point of 172-174°C. An NMR spectrum showed the product to have a structure consistent with 3-hexyl-7,7-diphenyl-2,4-dioxo-2,3,4,7-tetrahydro[1,3]oxazino[5',6':3,4]naphtho[1,2-b]pyran.

### EXAMPLE 12

The process of Example 9 was followed except that 5 grams of the naphthopyran were used and 2-methacryloyloxy ethyl isocyanate (10 mL) was used instead of methyl isocyanate and triethylamine was used instead of DABCO. A single product was isolated and had a melting point of 198-200°C. An NMR spectrum showed the product to have a structure consistent with 3-(2-methacryloyloxyethyl)-7,7-diphenyl-2,4-dioxo-2,3,4,7-tetrahydro[1,3]oxazino[5',6':3,4]naphtho[1,2-b]pyran.

### EXAMPLE 13

Benzophenone imine (10 grams) was added to a reaction flask containing tetrahydrofuran (THF) (100 mL). One equivalent of isopropyl magnesium bromide was slowly added to the mixture to produce a benzophenone imino magnesium bromide Grignard reagent. 2,2-Diphenyl-5-methoxycarbonyl-6-methoxy-2H-naphtho[1,2-b]pyran (3 grams) dissolved in 20 mL THF was added to the reagent mixture and stirred for two hours. A ten percent solution of hydrochloric acid (100 mL) was added and stirred for one half hour. The mixture was extracted with diethyl ether, washed, dried and concentrated. A solid product immediately formed in the concentrate. The solid product (Compound 13) was filtered, washed and dried and found to have a melting point of 274-276°C. The filtrate was passed through a silica column yielding a second desired product (Compound 13A) and a byproduct. NMR spectra and mass spectra showed Compound 13 (major product) to have a structure consistent with 2,2,7,7-tetraphenyl-4-oxo-2,3,4,7-tetrahydro-1H-pyrimidino[5',4':3,4]naphtho[1,2-b]pyran, Compound 13A (minor product) to have a structure consistent with 2,2,7,7-tetraphenyl-4-oxo-2,3,4,7-tetrahydro-1H-[1,3]oxazino[5',4':3,4]naphtho[1,2-b]pyran and the byproduct to have a structure consistent with 2,2-diphenyl-5-carbamoyl-6-methoxy-2H-naphtho[1,2-b]pyran.

### EXAMPLE 14

### Step 1

2,2-Diphenyl-5-methoxycarbonyl-6-methoxy-2H-naphtho[1,2-b]pyran (2 grams) dissolved in THF (10 mL) was slowly added to a reaction flask containing freshly prepared 2-methylpropylene-1-magnesium bromide, a 20% excess, and stirred for three hours. A 100 mL mixture of ice and a five percent solution of hydrochloric acid was added to the mixture and stirred for one half hour. The organic layer was separated, washed, dried and concentrated. The concentrate was purified by column chromatography using a 1:1 chloroform:hexane mixture as the eluant. The fractions with the desired product were collected and combined. The solvent was evaporated leaving 1.2 grams of an oily residue. An NMR spectrum showed the product to have a structure consistent with 2,2-diphenyl-5-methoxycarbonyl-6-(2-methylprop-1-enyl)-2H-naphtho[1,2-b]pyran.

### Step 2

One gram of the naphthopyran produced in Step 1, trimethyl silyl chloride (1 gram) and sodium iodide (1 gram) were added to a reaction flask containing anhydrous acetonitrile (30 mL) and stirred at 50°C for eight hours. Water (50 mL) was added to the mixture, and the mixture was extracted with diethyl ether. The ether layer was washed with 5% sodium thiosulfate solution followed by water. The washed ether layer was dried, concentrated and passed through a silica gel column. The fractions containing the desired product were collected and concentrated yielding 100 mg of an oily product. An NMR spectrum showed the product to have a structure consistent with 7,7-diphenyl-1,2,4,7-tetrahydro-2,2-dimethylpyrano[3',4':3,4]naphtho[1,2-b]pyran.

### EXAMPLE 15

### PART A

Testing was done with the photochromic compounds prepared in Examples 1-14 in the following manner. A quantity of each photochromic compound, except Compounds 13A and 14, calculated to yield a 1.5 x 10⁻³ molal solution was added to a flask containing 50 grams of a monomer blend of 4 parts ethoxylated bisphenol A dimethacrylate (BPA 2EO DMA), 1 part poly(ethylene glycol) 600 dimethacrylate, and 0.033 weight percent 2,2'-azobis(2-methyl propionitrile) (AIBN). Each photochromic compound was dissolved into the monomer blend by stirring and gentle heating. After a clear solution was obtained, it was poured into a flat sheet mold having the interior dimensions of 2.2 mm x 6 inches (15.24 cm) x 6 inches (15.24 cm). The mold was sealed and placed in a horizontal air flow, programmable oven programmed to increase the temperature from 40°C to 95°C over a 5 hour interval, hold the temperature at 95°C for 3 hours, lower it to 60°C over a 2 hour interval and then hold at 60°C for 16 hours. After the mold was opened, the polymer sheet was cut using a diamond blade saw into 2 inch (5.1 centimeters) test squares.

The compound of Example 13 identified as 13A and the compound of Example 14 were dissolved in diethylene glycol dimethyl ether. The concentration of the resulting solution was approximately 0.5 milligram per milliliter. Each solution was tested in a UV/Visible Spectrophotometer to determine the lambda max (Vis).

### Part B

The photochromic test squares prepared in Part A were tested for photochromic response on an optical bench. Prior to testing on the optical bench, the photochromic test squares were exposed to 365 nanometer ultraviolet light for about 15 minutes to activate the photochromic compounds and then placed in a 76°C oven for about 15 minutes to bleach or inactivate the photochromic compounds. The test squares were then cooled to room temperature, exposed to fluorescent room lighting for at least 2 hours and then kept covered for at least 2 hours prior to testing on an optical bench maintained at 72°F (22.2°C). The bench was fitted with a 150 watt Xenon arc lamp, a remote controlled shutter, a copper sulfate bath acting as a heat sink for the arc lamp, a Schott WG-320 nm cut-off filter which removes short wavelength radiation; neutral density filter(s) and a sample holder in which the square to be tested was inserted. The power output of the optical bench, i.e., the dosage of light that the sample lens would be exposed to, was calibrated with a photochromic test square used as a reference standard. This resulted in a power output ranging from 0.15 to 0.20 milliWatts per square centimeter (mW/cm²). Measurement of the power output was made using a GRASEBY Optronics Model S-371 portable photometer (Serial #21536) with a UV-A detector (Serial #22411) or comparable equipment. The UV-A detector was placed into the sample holder and the light output was measured. Adjustments to the power output were made by increasing or decreasing the lamp wattage or by adding or removing neutral density filters in the light path.

A monitoring, collimated beam of light from a tungsten lamp was passed through the square at a small angle (approximately 30°) normal to the square. After passing through the square, the light from the tungsten lamp was directed to a detector through Spectral Energy Corp. GM-200 monochromator set at the previously determined visible lambda max of the photochromic compound being measured. The output signals from the detector were processed by a radiometer.

Change in optical density (ΔOD) was determined by inserting a test square in the bleached state into the sample holder, adjusting the transmittance scale to 100%, opening the shutter from the Xenon lamp to provide ultraviolet radiation to change the test square from the bleached state to an activated (i.e., darkened) state, measuring the transmittance in the activated state, and calculating the change in optical density according to the formula: ΔOD=log(100/%Ta), where %Ta is the percent transmittance in the activated state and the logarithm is to the base 10.

The optical properties of the photochromic compound in the test squares are reported in Table 1. The Δ OD/min, which represents the sensitivity of the photochromic compound's response to UV light, was measured over the first five (5) seconds of UV exposure, then expressed on a per minute basis. The saturation optical density (Δ OD@ Saturation) was taken under identical conditions as the Δ OD/min, except UV exposure was continued for 15 minutes. The lambda max (Vis) is the wavelength in nanometers (nm) in the visible spectrum at which the maximum absorption of the activated (colored) form of the photochromic compound in a test square occurs. The lambda max (Vis) wavelength was determined by testing the photochromic test square polymerizates of Part A in a Varian Cary 3 UV-Visible spectrophotometer. The Bleach Rate (T 1/2) is the time interval in seconds for the absorbance of the activated form of the photochromic compound in the test squares to reach one half the highest absorbance at room temperature (72°F, 22.2°C) after removal of the source of activating light.

**Table 1**

| Compound Example | lambda max (Vis) nanometers | Sensitivity ΔOD/min | ΔOD @ Saturation | Bleach Rate T 1/2 (sec) |
|---|---|---|---|---|
| 1 | 472 | 0.76 | 1.89 | 220 |
| 2 | 510 | 0.74 | 0.67 | 51 |
| 3 | 496 | 0.75 | 1.28 | 117 |
| 4 | 474 | 0.67 | 1.71 | 248 |
| 5 | 478 | 0.67 | 1.74 | 254 |
| 6 | 475 | 0.64 | 1.72 | 240 |
| 7 | 476 | 0.60 | 1.46 | 248 |
| 8 | 475 | 0.63 | 1.58 | 251 |
| 9 | 469 | 0.41 | 0.40 | 51 |
| 10 | 468 | 0.44 | 0.38 | 42 |
| 11 | 471 | 0.46 | 0.41 | 53 |
| 12 | 467 | 0.26 | 0.32 | 140 |
| 13 | 512 | 0.62 | 2.45 | 533 |
| 13A | 471 | - | - | - |
| 14 | 460 | - | - | - |

The results of Table 1 show that test squares prepared using the Compounds of Examples 1 through 14 and the solutions of Compounds 13A and Example 14 demonstrate a range of colors from wavelengths of 460 nm to 512 nm, coloration rates (sensitivity) from 0.26 to 0.76, activated intensity (ΔOD at Saturation) from 0.40 to 2.45, and fade or bleach rates from 42 to 533 seconds.

The present invention has been described with reference to specific details of particular embodiments thereof. It is not intended that such details be regarded as limitations upon the scope of the invention except insofar as to the extent that they are included in the accompanying claims.

## Claims

1. A naphthopyran compound represented by the following graphic formula: 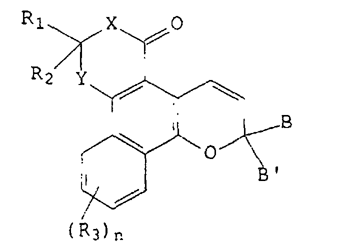 wherein,
(a) R₁ and R₂ together form an oxo group or R₁ is hydrogen and R₂ is hydrogen. C₁-C₆ alkyl, C₃-C₇ cycloalkyl, allyl, phenyl, mono-or di-substituted phenyl, benzyl, mono-substituted benzyl, naphthyl, mono- or di-substituted naphthyl, C₄-C₁₂ bicycloalkyl, linear or branched C₃-C₁₂ alkenyl, C₁-C₆ alkoxy carbonyl(C₁-C₆)alkyl, methacryloxy(C₁-C₆)alkyl, acryloxy(C₁-C₆)alkyl, C₁-C₄ acyloxy(C₁-C₆)alkyl, C₁-C₆ alkoxy(C₁-C₆)alkyl or the unsubstituted, mono- or di-substituted heteroaromatic groups pyridyl, furanyl, benzofuran-2-yl, benzyfuran-3-yl, thienyl, benzothien-2-yl, benzothien-3-yl, dibenzofuranyl, dibenzothienyl, carbazolyl, benzopyridyl and indolyl, each of said phenyl, benzyl, naphthyl and heteroaromatic group substituents being C₁-C₆ alkyl, C₁-C₆ alkoxy, morpholino, di(C₁-C₆)alkylamino, chloro or fluoro;
(b) R₃ is selected from the group consisting of C₁-C₆ alkyl, C₁-C₆ alkoxy, chloro, fluoro, phenyl, mono- and di-substituted phenyl, benzyl or mono-substituted benzyl, C₃-C₇ cycloalkyl, aryloxy, di(C₁-C₆)alkylamino, morpholino, thiomorpholino, piperidino, pyridyl, tetrahydroquinolino, isoquinolino, aziridino, diarylamino, N-(C₁-C₆)alkyl piperizino and N-aryl piperizino, wherein the aryl groups are phenyl or naphthyl, each of said phenyl and benzyl substituents being C₁-C₆ alkyl, C₁-C₆ alkoxy, fluoro and chloro, and n is the integer 0, 1 or 2;
(c) X is oxygen or -N(R₄)-, wherein R₄ is hydrogen C₁-C₆ alkyl, C₃-C₇ cycloalkyl, allyl, vinyl, C₁-C₅ acyl, phenyl, mono- and di-substituted phenyl, benzyl, mono-substituted benzyl, C₁-C₄ alkoxycarbonyl(C₁-C₆)alkyl, methacryloxy(C₁-C₆)alkyl, acryloyloxy(C₁-C₆)alkyl, phenyl(C₁-C₆)alkyl, naphthyl, C₄-C₁₂ bicycloalkyl, C₂-C₄ acyloxy or the unsubstituted or substituted heteroaromatic groups pyridyl, furanyl, benzofuran-2-yl, benzofuran-3-yl, thienyl, benzothien-2-yl, benzothien-3-yl, dibenzofuranyl, dibenzothienyl, carbazolyl, benzopyridyl and indolyl, each of said phenyl, benzyl and heteroaromatic group substituents being C₁-C₆ alkyl or C₁-C₆ alkoxy;
(d) Y is oxygen, -N(R₄)- or -C(R₅)R₆-, wherein R₅ and R₆ are each hydrogen, C₁-C₆ alkyl or C₃-C₇ cycloalkyl, with the proviso that when Y is -(C(R₅)R₆)-, X is oxygen; and
(e) B and B' are each selected from the group consisting of:
(i) the unsubstituted, mono-, di- and tri-substituted aryl groups, phenyl and naphthyl;
(ii) the unsubstituted, mono- and di-substituted heteroaromatic groups pyridyl, furanyl, benzofuran-2-yl, benzofuran-3-yl, thienyl, benzothien-2-yl, benzothien-3-yl, dibenzofuranyl, dibenzothienyl, carbazolyl, benzopyridyl, indoloyl and fluorenyl, each of said aryl and heteroaromatic substituents in parts (i) and (ii) being selected from the group consisting of hydroxy, aryl, i.e., phenyl and naphthyl, mono(C₁-C₆)alkoxyaryl, di(C₁-C₆)alkoxyaryl, mono(C₁-C₆)alkylaryl, di(C₁-C₆)alkylaryl, chloroaryl, fluoroaryl, C₃-C₇ cycloalkylaryl, C₃-C₇ cycloalkyl, C₃-C₇ cycloalkyloxy, C₃-C₇ cycloalkyloxy(C₁-C₆)alkyl, C₃-C₇ cycloalkyloxy(C₁-C₆)alkoxy, aryl(C₁-C₆)alkyl, aryl(C₁-C₆)alkoxy, aryloxy, aryloxy(C₁-C₆)alkyl, aryloxy(C₁-C₆)alkoxy, mono- and di-(C₁-C₆)alkylaryl(C₁-C₆)alkyl, mono-and di-(C₁-C₆)alkoxyaryl(C₁-C₆)alkyl, mono- and di-(C₁-C₆)alkylaryl(C₁-C₆)alkoxy. mono- and di-(C₁-C₆)alkoxyaryl(C₁-C₆)alkoxy, amino, mono(C₁-C₆)alkylamino, di(C₁-C₆)alkylamino, diarylamino, piperazino, N-(C₁-C₆)alkylpiperazino, N-arylpiperazino, aziridino, indolino, piperidino, morpholino, thiomorpholino, tetrahydroquinolino, tetrahydroisoquinolino, pyrryl, C₁-C₆ alkyl, C₁-C₆ chloroalkyl, C₁-C₆ fluoroalkyl, C₁-C₆ alkoxy, mono(C₁-C₆)alkoxy(C₁-C₄)alkyl, acryloxy, methacryloxy, bromo, chloro and fluoro;
(iii) the groups represented by the following graphic formulae: 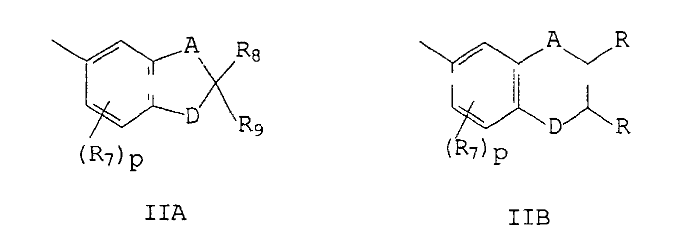 wherein A may be carbon or oxygen and D may be oxygen or substituted nitrogen, provided that when D is substituted nitrogen, A is carbon, said nitrogen substituents being selected from the group consisting of hydrogen, C₁-C₆ alkyl and C₂-C₆ acyl; each R₇ is C₁-C₆ alkyl, C₁-C₆ alkoxy, hydroxy, chloro or fluoro; R₈ and R₉ are each hydrogen or C₁-C₆ alkyl; and p is the integer 0, 1 or 2;
(iv) C₁-C₆ alkyl, C₁-C₆ chloroalkyl, C₁-C₆ fluoroalkyl, C₁-C₆ alkoxy(C₁-C₄)alkyl, C₃-C₆ cycloalkyl, mono(C₁-C₆)alkoxy(C₃-C₆)cycloalkyl, mono(C₁-C₆)alkyl(C₃-C₆)cycloalkyl, chloro(C₃-C₆)cycloalkyl, fluoro(C₃-C₆)cycloalkyl and C₄-C₁₂ bicycloalkyl; and
(v) the group represented by the following graphic formula: 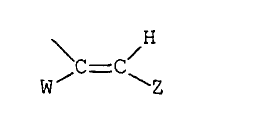 wherein W may be hydrogen or C₁-C₄ alkyl and Z may be selected from the unsubstituted, mono-, and di-substituted members of the group consisting of naphthyl, phenyl, furanyl and thienyl, each of said group substituents in this part (v) being C₁-C₄ alkyl, C₁-C₄ alkoxy, fluoro or chloro; or
(vi) B and B' taken together form fluoren-9-ylidene. mono-, or di-substituted fluoren-9-ylidene or form a member selected from the group consisting of saturated C₃-C₁₂ spiro-monocyclic hydrocarbon rings, saturated C₇-C₁₂ spirobicylic hydrocarbon rings, and saturated C₇-C₁₂ spiro-tricyclic hydrocarbon rings, each of said fluoren-9-ylidene substituents being selected from the group consisting of C₁-C₄ alkyl, C₁-C₄ alkoxy, fluoro and chloro.

2. The naphthopyran of claim 1 wherein,
(a) R₁ is hydrogen and R₂ is hydrogen, C₁-C₅ alkyl, C₃-C₆ cycloalkyl, phenyl, mono- or di-substituted phenyl, benzyl or mono-substituted benzyl, each of said phenyl and benzyl group substituents being C₁-C₄ alkyl or C₁-C₄ alkoxy;
(b) R₃ is selected from the group consisting of C₁-C₄ alkyl, C₁-C₄ alkoxy, fluoro, phenyl and aryloxy, and n is the integer 0, 1, or 2;
(c) X is oxygen or -N(R₄)-, wherein R₄ is hydrogen, C₁-C₃ alkyl, methacryloxy(C₁-C₆)alkyl or acryloxy(C₁-C₆)alkyl;
(d) Y is oxygen, -NH- or -CH₂-; and
(e) B and B' are each selected from the group consisting of:
(i) phenyl, mono-substituted phenyl and di-substituted phenyl;
(ii) the unsubstituted, mono- and di-substituted heteroaromatic groups furanyl, benzofuran-2-yl, thienyl, benzothien-2-yl, dibenzofuranyl, aryloxy and diarylamino, each of said phenyl and heteroaromatic substituents being selected from the group consisting of di(C₁-C₃)alkylamino, piperidino, morpholino, pyrryl, C₁-C₃ alkyl, C₁-C₃ chloroalkyl, C₁-C₃ fluoroalkyl, C₁-C₃ alkoxy, mono(C₁-C₃)alkoxy(C₁-C₃)alkyl, fluoro and chloro;
(iii) the groups represented by the graphic formulae IIA and IIB, wherein A is carbon and D is oxygen, R₇ is C₁-C₃ alkyl or C₁-C₃ alkoxy, R₈ and R₉ are each hydrogen or C₁-C₄ alkyl, and p is the integer 0 or 1;
(iv) C₁-C₄ alkyl; and
(v) the group represented by the graphic formula IIC wherein W is hydrogen or methyl and Z is phenyl or mono-substituted phenyl, said phenyl substituent being selected from the group consisting of C₁-C₃ alkyl, C₁-C₃ alkoxy and fluoro; or
(vi) B and B' taken together form fluoren-9-ylidene, mono-substituted fluoren-9-ylidene or a member selected from the group consisting of saturated C₃-C₈ spiro-monocyclic hydrocarbon rings, saturated C₇-C₁₀ spiro-bicyclic hydrocarbon rings, and saturated C₇-C₁₀ spiro-tricyclic hydrocarbon rings, said fluoren-9-ylidene substituent being selected from the group consisting of C₁-C₃ alkyl, C₁-C₃ alkoxy, fluoro and chloro.

3. The naphthopyran of claim 2 wherein,
(a) R₁ is hydrogen and R₂ is hydrogen, C₁-C₅ alkyl or phenyl:
(b) R₃ is selected from the group consisting of C₁-C₃ alkyl, C₁-C₃ alkoxy, phenyl and aryloxy and n is the integer 0, 1, or 2;
(c) B and B' are each selected from the group consisting of:
(i) phenyl, mono- and di-substituted phenyl;
(ii) the unsubstituted, mono- and di-substituted heteroaromatic groups furanyl, benzofuran-2-yl, thienyl, benzothien-2-yl, dibenzofuranyl, aryloxy and diarylamino, each of said phenyl and heteroaromatic substituents being selected from the group consisting of C₁-C₃ alkyl, C₁-C₃ alkoxy and fluoro; and
(iii) the group represented by graphic formula IIA, wherein A is carbon and D is oxygen, R₇ is C₁-C₃ alkyl or C₁-C₃ alkoxy, R₈ and R₉ are each hydrogen or C₁-C₃ alkyl, and p is the integer 0 or 1; or
(iv) B and B' taken together form fluoren-9-ylidene, adamantylidene, bornylidene, norbornylidene, or bicyclo[3.3.1]nonan-9-ylidene.

4. A naphthopyran compound selected from the group consisting of:
a) 7,7-diphenyl-4-oxo-4H-7H-[1,3]dioxino[5',4':3,4]naphtho[1,2-b]pyran;
b) 7,7-di(4-methoxyphenyl)-4-oxo-4H-7H-[1,3]dioxino[5',4':3,4]naphtho[1,2-b]pyran;
c) 7-(4-methoxyphenyl)-7-phenyl-2-pentyl-4-oxo-4H-7H-[1,3]dioxino[5',4':3,4]naphtho[1,2-b]pyran;
d) 7,7-diphenyl-2-ethyl-4-oxo-4H-7H-[1,3]dioxino[5',4':3,4]naphtho[1,2-b]pyran;
e) 7,7-diphenyl-2-pentyl-4-oxo-4H-7H-[1,3]dioxino[5',4':3,4]naphtho[1,2-b]pyran;
f) 7,7-diphenyl-2-(2-methylpropyl)-4-oxo-4H-7H-[1,3]dioxino[5',4':3,4]naphtho[1,2-b]pyran;
g) 2,7,7-triphenyl-4-oxo-4H-7H-[1,3]dioxino[5',4':3,4]naphtho[1,2-b]pyran;
h) 7,7-diphenyl-2-(1-phenylethyl)-4-oxo-4H-7H-[1,3]dioxino[5',4';3,4]naphtho[1,2-b]pyran;
i) 3-methyl-7,7-diphenyl-2,4-dioxo-2,3,4,7-tetrahydro[1,3]oxazino[5',6':3,4]naphtho[1,2-b]pyran:
j) 3-(2-ethoxycarbonylethyl)-7,7-diphenyl-2,4-dioxo-2,3,4,7-tetrahydro[1,3]oxazino[5',6':3,4]naphtho[1,2-b]pyran;
k) 3-hexyl-7,7-diphenyl-2,4-dioxo-2,3,4,7-tetrahydro[1,3]oxazino[5',6':3,4]naphtho[1,2-b]pyran;
l) 3-(2-methacryloyloxyethyl)-7,7-diphenyl-2,4-dioxo-2,3,4,7-tetrahydro[1,3]oxazino[5',6':3,4]naphtho[1,2-b]pyran;
m) 2,2,7,7-tetraphenyl-4-oxo-2,3,4,7-tetrahydro-1H-pyrimidino[5',4':3,4]naphtho[1,2-b]pyran;
n) 2,2,7,7-tetraphenyl-4-oxo-2,3,4,7-tetrahydro-1H-[1,3]oxazino[5',4':3,4]naphtho[1,2-b]pyran; and
o) 7,7-diphenyl-1,2,4,7-tetrahydro-2,2-dimethylpyrano[3',4':3,4]naphtho[1,2-b]pyran.

5. A photochromic article comprising a polymeric organic host material and a photochromic amount of the naphthopyran compound of any of claims claim 1-4.

6. A photochromic article comprising, in combination, a solid transparent polymeric organic host material, and a photochromic amount of each of (a) at least one naphthopyran compound of claim 1-4, and (b) at least one other organic photochromic compound having at least one activated absorption maxima within the range of between about 400 and 700 nanometers.

7. The photochromic article of claim 6 wherein the organic photochromic compound (b) is selected from the group consisting of other naphthopyrans, chromenes, oxazines, metal-dithizonates, fulgides and fulgimides.

8. The photochromic article of any of claims 5-7 wherein the total amount of photochromic compound present is from 0.05 to 1.0 milligram per square centimeter of organic host material surface to which the photochromic substance(s) is incorporated or applied.

9. The photochromic article of any of claims 5-7 wherein the polymeric organic host material is selected from the group consisting of poly(C₁-C₁₂ alkyl methacrylates), poly(oxyalkylene dimethacrylates), poly(alkoxylated phenol methacrylates), cellulose acetate, cellulose triacetate, cellulose acetate propionate, cellulose acetate butyrate, poly(vinyl acetate), poly(vinyl alcohol), poly(vinyl chloride), poly(vinylidene chloride), thermoplastic polycarbonates, polyesters, polyurethanes, poly(ethylene terephthalate), polystyrene, poly(alpha methyl styrene), copoly(styrene-methylmethacrylate), copoly(styrene-acrylonitrile), polyvinylbutyral and polymers of members of the group consisting of bis(allyl carbonate) monomers, polyfunctional acrylate monomers, polyfunctional methacrylate monomers, diethylene glycol dimethacrylate monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers, alkoxylated polyhydric alcohol acrylate monomers, styrene monomers, urethane acrylate monomers, glycidyl acrylate monomers, glycidyl methacrylate monomers and diallylidene pentaerythritol monomers.

10. The photochromic article of claim 9 wherein the polymeric organic host material is a solid transparent polymer selected from the group consisting of poly(methyl methacrylate), poly(ethylene glycol bismethacrylate), poly(ethoxylated bisphenol A dimethacrylate), thermoplastic polycarbonate, poly(vinyl acetate), polyvinylbutyral, polyurethane and polymers of members of the group consisting of diethylene glycol bis(allyl carbonate) monomers, diethylene glycol dimethacrylate monomers, ethoxylated phenol bismethacrylate monomers, diisopropenyl benzene monomers and ethoxylated trimethylol propane triacrylate monomers.

11. The photochromic article of any of claims 5-7 wherein the host material is a polymerizate of an optical organic resin monomer.

12. The photochromic article of claim 11 wherein the refractive index of the polymerizate is from about 1.48 to about 1.75.

13. The photochromic article of claim 12 wherein the refractive index of the polymerizate is from about 1.495 to about 1.66.

14. The photochromic article of any of claims 5-13 wherein said article is an optical element.

15. The photochromic article of claim 14 wherein said optical element is a lens.

## Patentansprüche

1. Naphthopyranverbindung, dargestellt durch die folgende Strukturformel: 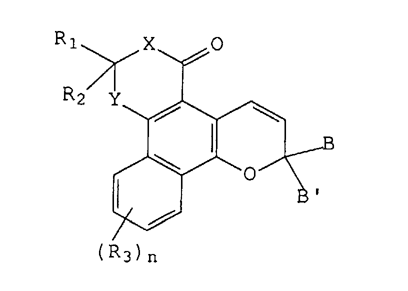 wobei:
(a) R₁ und R₂ zusammen eine Oxogruppe bilden oder R₁ Wasserstoff ist und R₂ Wasserstoff, C₁-C₆-Alkyl, C₃-C₇-Cycloalkyl, Allyl, Phenyl, mono- oder disubstituiertes Phenyl, Benzyl, monosubstituiertes Benzyl, Naphthyl, mono- oder disubstituiertes Naphthyl, C₄-C₁₂-Bicycloalkyl, lineares oder verzweigtes C₃-C₁₂-Alkenyl, C₁-C₆-Alkoxycarbonyl(C₁-C₆)alkyl, Methacryloxy(C₁-C₆)alkyl, Acryloxy-(C₁-C₆)alkyl, C₁-C₄-Acyloxy(C₁-C₆)alkyl, C₁-C₆ Alkoxy-(C₁-C₆)alkyl oder die unsubstituierten, mono- oder disubstituierten heteroaromatischen Gruppen Pyridyl, Furanyl, Benzofuran-2-yl, Benzyfuran-3-yl, Thienyl, Benzothien-2-yl, Benzothien-3-yl, Dibenzofuranyl, Dibenzothienyl, Carbazolyl, Benzopyridyl und Indolyl ist, wobei die Substituenten der besagten Phenyl-, Benzyl-, Naphthyl- und heteroaromatischen Gruppen C₁-C₆-Alkyl, C₁-C₆-Alkoxy, Morpholino, Di(C₁-C₆)alkylamino, Chlor oder Fluor darstellen;
(b) R₃ ausgewählt ist aus der Gruppe bestehend aus C₁-C₆-Alkyl, C₁-C₆-Alkoxy, Chlor, Fluor, Phenyl, mono- und disubstituiertem Phenyl, Benzyl oder monosubstituiertem Benzyl, C₃-C₇-Cycloalkyl, Aryloxy, Di(C₁-C₆)alkylamino, Morpholino, Thiomorpholino, Piperidino, Pyridyl, Tetrahydrochinolino, Isochinolino, Aziridino, Diarylamino, N-(C₁-C₆)Alkylpiperazino and N-Arylpiperazino, wobei die Arylgruppen Phenyl oder Naphthyl darstellen, jeder von genannten Phenylund Benzylsubstituenten C₁-C₆-Alkyl, C₁-C₆-Alkoxy, Fluor und Chlor ist und n die ganze Zahl 0, 1 oder 2 ist;
(c) X Sauerstoff oder -N(R₄)- ist, wobei R₄ Wasserstoff, C₁-C₆-Alkyl, C₃-C₇-Cycloalkyl, Allyl, Vinyl, C₁-C₅-Acyl, Phenyl, mono- und disubstituiertes Phenyl, Benzyl, monosubstituiertes Benzyl, C₁-C₄-Alkoxycarbonyl(C₁-C₆)alkyl, Methacryloxy(C₁-C₆)alkyl, Acryloyloxy(C₁-C₆)alkyl, Phenyl(C₁-C₆)-alkyl, Naphthyl, C₄-C₁₂-Bicycloalkyl, C₂-C₄-Acyloxy oder die unsubstituierten oder substituierten heteroaromatischen Gruppen Pyridyl, Furanyl, Benzofuran-2-yl, Benzofuran-3-yl, Thienyl, Benzothien-2-yl, Benzothien-3-yl, Dibenzofuranyl, Dibenzothienyl, Carbazolyl, Benzopyridyl und Indolyl darstellt, wobei die Substituenten jeder der genannten Phenyl-, Benzyl- und heteroaromatischen Gruppen C₁-C₆-Alkyl oder C₁-C₆-Alkoxy sind;
(d) Y Sauerstoff, -N(R4)- oder -C(R₅)R₆- ist, wobei R₅ und R₆ jeweils Wasserstoff, C₁-C₆-Alkyl oder C₃-C₇-Cycloalkyl sind, unter der Bedingung, dass, wenn Y -(C(R₅)R₆)- ist, X Sauerstoff ist; und
(e) B und B' jeweils ausgewählt sind aus der Gruppe bestehend aus:
(i) den unsubstituierten, mono-, di- und trisubstituierten Arylgruppen, Phenyl und Naphthyl;
(ii) den unsubstituierten, mono- und disubstituierten heteroaromatischen Gruppen Pyridyl, Furanyl, Benzofuran-2-yl, Benzofuran-3-yl, Thienyl, Benzothien-2-yl, Benzothien-3-yl, Dibenzofuranyl, Dibenzothienyl, Carbazolyl, Benzopyridyl, Indoloyl und Fluorenyl, wobei jeder von besagten Aryl- und heteroaromatischen Substituenten in den Teilen (i) und (ii) ausgewählt ist aus der Gruppe bestehend aus Hydroxy, Aryl, d.h. Phenyl und Naphthyl, Mono(C₁-C₆)-alkoxyaryl, Di(C₁-C₆)alkoxyaryl, Mono(C₁-C₆)alkylaryl, Di(C₁-C₆)alkylaryl, Chloraryl, Fluoraryl, C₃-C₇-Cycloalkylaryl, C₃-C₇-Cycloalkyl, C₃-C₇-Cycloalkyloxy, C₃-C₇-Cycloalkyloxy(C₁-C₆)alkyl, C₃-C₇-Cycloalkyloxy(C₁-C₆)alkoxy, Aryl(C₁-C₆)alkyl, Aryl(C₁-C₆)alkoxy, Aryloxy, Aryloxy-(C₁-C₆)alkyl, Aryloxy(C₁-C₆)alkoxy, Mono- und Di(C₁-C₆)-alkylaryl(C₁-C₆)alkyl, Mono- und Di(C₁-C₆)alkoxyaryl-(C₁-C₆)alkyl, Mono- und Di(C₁-C₆)alkylaryl(C₁-C₆)alkoxy, Mono- und Di(C₁-C₆)alkoxyaryl(C₁-C₆)alkoxy, Amino, Mono(C₁-C₆)alkylamino, Di(C₁-C₆)alkylamino, Diarylamino, Piperazino, N-(C₁-C₆)Alkylpiperazino, N-Arylpiperazino, Aziridino, Indolino, Piperidino, Morpholino, Thiomorpholino, Tetrahydrochinolino, Tetrahydroisochinolino, Pyrryl, C₁-C₆-Alkyl, C₁-C₆-Chloralkyl, C₁-C₆-Fluoralkyl, C₁-C₆-Alkoxy, Mono(C₁-C₆)alkoxy(C₁-C₄)alkyl, Acryloxy, Methacryloxy, Brom, Chlor und Fluor;
(iii) den Gruppen, dargestellt durch die folgenden Strukturformeln: 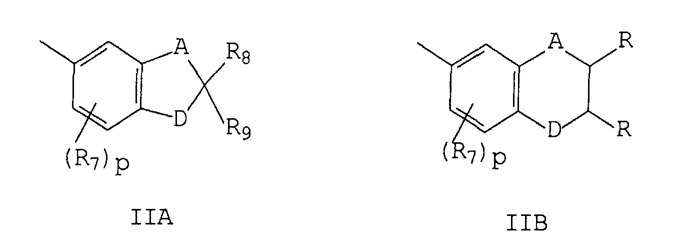 wobei A Kohlenstoff oder Sauerstoff sein kann und D Sauerstoff oder substituierter Stickstoff sein kann, unter der Bedingung, dass, wenn D substituierter Stickstoff ist, A Kohlenstoff ist, wobei besagte Stickstoffsubstituenten ausgewählt sind aus der Gruppe bestehend aus Wasserstoff, C₁-C₆-Alkyl und C₂-C₆-Acyl; jedes R₇ C₁-C₆-Alkyl, C₁-C₆-Alkoxy, Hydroxy, Chlor oder Fluor ist; R₈ und R₉ jeweils Wasserstoff oder C₁-C₆-Alkyl sind und p die ganze Zahl 0, 1 oder 2 ist:
(iv) C₁-C₆-Alkyl, C₁-C₆-Chloralkyl, C₁-C₆-Fluoralkyl, C₁-C₆-Alkoxy(C₁-C₄)alkyl, C₃-C₆-Cycloalkyl, Mono(C₁-C₆)-alkoxy(C₃-C₆)cycloalkyl, Mono(C₁-C₆)alkyl(C₃-C₆)cycloalkyl. Chlor(C₃-C₆)cycloalkyl, Fluor(C₃-C₆)cycloalkyl und C₄-C₁₂-Bicycloalkyl und
(v) der Gruppe, dargestellt durch die folgende Strukturformel: 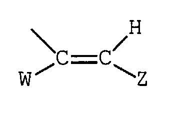 wobei W Wasserstoff oder C₁-C₄-Alkyl sein kann und Z ausgewählt sein kann aus den unsubstituierten, mono- und disubstituierten Mitgliedern der Gruppe bestehend aus Naphthyl, Phenyl, Furanyl und Thienyl, wobei jeder der Substituenten der besagten Gruppen in diesem Teil (v) C₁-C₄-Alkyl, C₁-C₄-Alkoxy, Fluor oder Chlor ist oder
(vi) B und B' zusammengenommen Fluoren-9-yliden, monooder disubstitutiertes Fluoren-9-yliden bilden oder ein Mitglied, ausgewählt aus der Gruppe bestehend aus gesättigten C₃-C₁₂-spiromonocyclischen Kohlenwasserstoffringen, gesättigten C₇-C₁₂-spirobicylischen Kohlenwasserstoffringen und gesättigten C₇-C₁₂-spirotricyclischen Kohlenwasserstoffringen, bilden, wobei jeder von besagten Fluoren-9-ylidensubstituenten ausgewählt ist aus der Gruppe bestehend aus C₁-C₄-Alkyl, C₁-C₄-Alkoxy, Fluor und Chlor.

2. Naphthopyran nach Anspruch 1, wobei
(a) R₁ Wasserstoff ist und R₂ Wasserstoff, C₁-C₅-Alkyl, C₃-C₆-Cycloalkyl, Phenyl, mono- oder disubstituiertes Phenyl, Benzyl oder monosubstituiertes Benzyl ist, wobei jeder von besagten Phenyl- und Benzylgruppensubstituenten C₁-C₄-Alkyl oder C₁-C₄-Alkoxy ist;
(b) R₃ ausgewählt ist aus der Gruppe bestehend aus C₁-C₄-Alkyl, C₁-C₄-Alkoxy, Fluor, Phenyl und Aryloxy und n die ganze Zahl 0, 1 oder 2 ist;
(c) X Sauerstoff oder -N(R₄)- ist, wobei R₄ Wasserstoff, C₁-C₃-Alkyl, Methacryloxy(C₁-C₆)alkyl oder Acryloxy(C₁-C₆)alkyl ist;
(d) Y Sauerstoff, -NH- oder -CH₂- ist und
(e) B und B' jeweils ausgewählt sind aus der Gruppe bestehend aus:
(i) Phenyl, monosubstituiertem Phenyl und disubstituiertem Phenyl;
(ii) den unsubstituierten, mono- und disubstituierten heteroaromatischen Gruppen Furanyl, Benzofuran-2-yl, Thienyl, Benzothien-2-yl, Dibenzofuranyl, Aryloxy und Diarylamino, wobei jeder von besagten Phenyl- und heteroaromatischen Substituenten ausgewählt ist aus der Gruppe bestehend aus Di(C₁-C₃)alkylamino, Piperidino, Morpholino, Pyrryl, C₁-C₃-Alkyl, C₁-C₃-Chloralkyl, C₁-C₃-Fluoralkyl, C₁-C₃-Alkoxy, Mono(C₁-C₃)alkoxy(C₁-C₃)alkyl, Fluor und Chlor;
(iii) den Gruppen, dargestellt durch die Strukturformeln IIA und IIB, wobei A Kohlenstoff ist und D Sauerstoff ist, R₇ C₁-C₃-Alkyl oder C₁-C₃-Alkoxy ist, R₈ und R₉ jeweils Wasserstoff oder C₁-C₄-Alkyl sind und p die ganze Zahl 0 oder 1 ist;
(iv) C₁-C₄-Alkyl und
(v) der Gruppe, dargestellt durch die Strukturformel IIC, wobei W Wasserstoff oder Methyl ist und Z Phenyl oder monosubstituiertes Phenyl darstellt, wobei besagter Phenylsubstituent ausgewählt ist aus der Gruppe bestehend aus C₁-C₃-Alkyl, C₁-C₃-Alkoxy und Fluor, oder
(vi) B und B' zusammengenommen Fluoren-9-yliden, monosubstituiertes Fluoren-9-yliden bilden oder ein Mitglied, ausgewählt aus der Gruppe bestehend aus gesättigten C₃-C₈-spiromonocyclischen Kohlenwasserstoffringen, gesättigten C₇-C₁₀-spirobicyclischen Kohlenwasserstoffringen und gesättigten C₇-C₁₀-spirotricyclischen Kohlenwasserstoffringen, wobei besagter Fluoren-9-ylidensubstituent ausgewählt ist aus der Gruppe bestehend aus C₁-C₃-Alkyl, C₁-C₃-Alkoxy, Fluor und Chlor.

3. Naphthopyran nach Anspruch 2, wobei
(a) R₁ Wasserstoff ist und R₂ Wasserstoff, C₁-C₅-Alkyl oder Phenyl ist;
(b) R₃ ausgewählt ist aus der Gruppe bestehend aus C₁-C₃-Alkyl, C₁-C₃-Alkoxy, Phenyl und Aryloxy, und n die ganze Zahl 0, 1 oder 2 ist;
(c) B und B' jeweils ausgewählt sind aus der Gruppe bestehend aus:
(i) Phenyl, mono- und disubstituiertem Phenyl;
(ii) den unsubstituierten, mono- und disubstituierten heteroaromatischen Gruppen Furanyl, Benzofuran-2-yl, Thienyl, Benzothien-2-yl, Dibenzofuranyl, Aryloxy und Diarylamino, wobei jeder von besagten Phenyl- und heteroaromatischen Substituenten ausgewählt ist aus der Gruppe bestehend aus C₁-C₃-Alkyl, C₁-C₃-Alkoxy und Fluor, und
(iii) der Gruppe, dargestellt durch die Strukturformel IIA, wobei A Kohlenstoff und D Sauerstoff ist, R₇ C₁-C₃-Alkyl oder C₁-C₃-Alkoxy darstellt, R₈ und R₉ jeweils Wasserstoff oder C₁-C₃-Alkyl sind und p die ganze Zahl 0 oder 1 ist oder
(iv) B und B' zusammengenommen Fluoren-9-yliden, Adamantyliden, Bornyliden, Norbornyliden oder Bicyclo[3.3.1]nonan-9-yliden bilden.

4. Naphthopyranverbindung, ausgewählt aus der Gruppe bestehend aus:
a) 7,7-Diphenyl-4-oxo-4H-7H-[1,3]dioxino[5',4':3,4]-naphtho[1,2-b]pyran;
b) 7,7-Di(4-methoxyphenyl)-4-oxo-4H-7H-[1,3]dioxino-[5',4':3,4]naphtho[1,2-b]pyran;
c) 7-(4-Methoxyphenyl)-7-phenyl-2-pentyl-4-oxo-4H-7H-[1,3]dioxino[5',4':3,4]naphtho[1,2-b]pyran;
d) 7,7-Diphenyl-2-ethyl-4-oxo-4H-7H-[1,3]dioxino-[5',4':3,4]naphtho[1,2-b]pyran;
e) 7,7-Diphenyl-2-pentyl-4-oxo-4H-7H-[1,3]dioxino-[5',4':3,4]naphtho[1,2-b]pyran;
f) 7,7-Diphenyl-2-(2-methylpropyl)-4-oxo-4H-7H-[1,3]dioxino-[5',4':3,4]naphtho[1,2-b]pyran;
g) 2,7,7-Triphenyl-4-oxo-4H-7H-[1,3]dioxino[5',4':3,4]-naphtho[1,2-b]pyran;
h) 7,7-Diphenyl-2-(1-phenylethyl)-4-oxo-4H-7H-[1,3]dioxino-[5',4':3,4]naphtho[1,2-b]pyran;
i) 3-Methyl-7,7-diphenyl-2,4-dioxo-2,3,4,7-tetrahydro[1,3]-oxazino[5',6':3,4]naphtho[1,2-b]pyran;
j) 3-(2-Ethoxycarbonylethyl)-7,7-diphenyl-2,4-dioxo-2,3,4,7-tetrahydro[1,3]oxazino[5',6':3,4]naphtho[1,2-b]pyran;
k) 3-Hexyl-7,7-diphenyl-2,4-dioxo-2,3,4,7-tetrahydro-[1,3]oxazino[5',6':3,4]naphtho[1,2-b]pyran;
l) 3-(2-Methacryloyloxyethyl)-7,7-diphenyl-2,4-dioxo-2,3,4,7-tetrahydro[1,3]oxazino[5',6':3,4]naphtho[1,2-b]pyran;
m) 2,2,7,7-Tetraphenyl-4-oxo-2,3,4,7-tetrahydro-1Hpyrimidino[5',4':3,4]naphtho[1,2-b]pyran;
n) 2,2,7,7-Tetraphenyl-4-oxo-2,3,4,7-tetrahydro-1H-[1,3]oxazino[5'.4':3,4]naphtho[1,2-b]pyran und
o) 7,7-Diphenyl-1,2,4,7-tetrahydro-2,2-dimethylpyrano-[3',4':3,4]naphtho[1,2-b]pyran.

5. Photochromer Gegenstand, enthaltend ein polymeres organisches Wirtsmaterial und eine photochrome Menge der Naphthopyranverbindung nach einem der Ansprüche 1-4.

6. Photochromer Gegenstand, enthaltend, in Kombination, ein festes transparentes polymeres organisches Wirtsmaterial und eine photochrome Menge von jeweils (a) mindestens einer Naphthopyranverbindung nach Anspruch 1-4 und (b) mindestens einer anderen organischen photochromen Verbindung mit mindestens einem aktivierten Absorptionsmaximum innerhalb des Bereichs zwischen etwa 400 und 700 nm.

7. Photochromer Gegenstand nach Anspruch 6, wobei die organische photochrome Verbindung (b) ausgewählt ist aus der Gruppe bestehend aus anderen Naphthopyranen, Chromenen, Oxazinen, Metalldithiozonaten, Fulgiden und Fulgimiden.

8. Photochromer Gegenstand nach einem der Ansprüche 5-7, wobei die Gesamtmenge der vorliegenden photochromen Verbindung 0,05 bis 1,0 mg/cm² der organischen Wirtsmaterialoberfläche, in die die photochrome Substanz oder die photochromen Substanzen eingebracht oder auf diese aufgebracht sind, beträgt.

9. Photochromer Gegenstand nach einem der Ansprüche 5-7, wobei das polymere organische Wirtsmaterial ausgewählt ist aus der Gruppe bestehend aus Poly(C₁-C₁₂-alkylmethacrylaten), Poly(oxyalkylendimethacrylaten), Poly(alkoxylierten Phenolmethacrylaten), Celluloseacetat, Cellulosetriacetat, Celluloseacetatpropionat, Celluloseacetatbutyrat, Poly(vinylacetat), Poly(vinylalkohol), Poly(vinylchlorid), Poly(vinylidenchlorid), thermoplastischen Polycarbonaten, Polyestern, Polyurethanen, Poly(ethylenterephthalat), Polystyrol, Poly(alpha-methylstyrol), Copoly-(styrol-methylmethacrylat), Copoly(styrol-acrylonitril), Polyvinylbutyral und Polymeren der Mitglieder der Gruppe bestehend aus Bis(allylcarbonat)monomeren, polyfunktionellen Acrylatmonomeren, polyfunktionellen Methacrylatmonomeren, Diethylenglykoldimethacrylatmonomeren, Diisopropenylbenzolmonomeren, ethoxylierten Bisphenol-A-Dimethacrylatmonomeren, Ethylenglykolbismethacrylatmonomeren, Poly(ethylenglykol)bis(methacrylat)monomeren, ethoxylierten Phenolbismethacrylatmonomeren, alkoxylierten mehrere Hydroxylgruppen enthaltenden Alkoholacrylatmonomeren, Styrolmonomeren, Urethanacrylatmonomeren, Glycidylacrylatmonomeren, Glycidylmethacrylatmonomeren und Diallylidenpentaerythritolmonomeren.

10. Photochromer Gegenstand nach Anspruch 9, wobei das polymere organische Wirtsmaterial ein festes transparentes Polymer, ausgewählt aus der Gruppe bestehend aus Poly(methylmethacrylat), Poly(ethylenglykolbismethacrylat), poly(ethoxyliertem Bisphenol-A-Dimethacrylat), thermoplastischem Polycarbonat, Poly(vinylacetat), Polyvinylbutyral, Polyurethan und Polymeren von Mitgliedern der Gruppe bestehend aus Diethylenglykolbis(allylcarbonat)monomeren, Diethylenglykoldimethacrylatmonomeren, ethoxylierten Phenolbismethacrylatmonomeren, Diisopropenylbenzolmonomeren und ethoxylierten Trimethylolpropantriacrylatmonomeren ist.

11. Photochromer Gegenstand nach einem der Ansprüche 5-7, wobei das Wirtsmaterial ein Polymerisat eines optischen organischen Harzmonomeren ist.

12. Photochromer Gegenstand nach Anspruch 11, wobei der Brechungsindex des Poylmerisats etwa 1,48 bis etwa 1,75 betrügt.

13. Photochromer Gegenstand nach Anspruch 12, wobei der Brechungsindex des Poylmerisats etwa 1,495 bis etwa 1,66 betrügt.

14. Photochromer Gegenstand nach einem der Ansprüche 5-13, wobei besagter Gegenstand ein optisches Element darstellt.

15. Photochromer Gegenstand nach Anspruch 14, wobei besagtes optisches Element eine Linse ist.

## Revendications

1. Composé de naphtopyranne représenté par la formule graphique suivante : 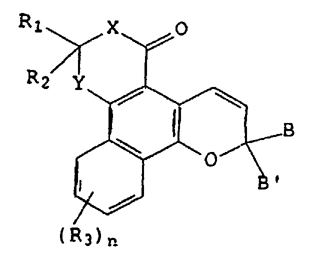 dans laquelle:
(a) R₁ et R₂ forment ensemble un groupe oxo ou bien R₁ est de l'hydrogène et R₂ est de l'hydrogène ou un groupe alkyle en C₁-C₆, cycloalkyle en C₃-C₇, allyle, phényle, phényle mono- ou disubstitué, benzyle, benzyle monosubstitué, naphtyle, naphtyle mono- ou disubstitué, bicycloalkyle en C₄-C₁₂, alcényle en C₃-C₁₂ linéaire ou ramifié, alcoxy (C₁-C₆)carbonylalkyle en C₁-C₆, méthacryloxyalkyle en C₁-C₆, acryloxyalkyle en C₁-C₆, acyloxy(C₁-C₄)alkyle en C₁-C₆, alcoxy(C₁-C₆)alkyle en C₁-C₆ ou les groupes hétéroaromatiques non substitués, mono- ou disubstitués pyridyle, furannyle, benzofurann-2-yle, benzylfurann-3-yle, thiényle, benzothén-2-yle, benzothién-3-yle, dibenzofurannyle, dibenzothiényle, carbazolyle, benzopyridyle et indolyle, chacun desdits substituants des groupes phényle, benzyle, naphtyle et hétéroaromatiques étant un groupe alkyle en C₁-C₆, alcoxy en C₁-C₆, morpholino, dialkylamino en C₁-C₆, chloro ou fluoro;
(b) R₃ est choisi dans le groupe comprenant les groupes alkyle en C₁-C₆, alcoxy en C₁-C₆, chloro, fluoro, phényle, phényle mono- et disubstitués, benzyle et benzyle monosubstitués, cycloalkyle en C₃-C₇, aryloxy, dialkylamino en C₁-C₆, morpholino, thiomorpholino, pipéridino, pyridyle, tétrahydroquinoléino, isoquinoléino, aziridino, diarylamino, N-alkyl(C₁-C₆) pipérizino et N-aryl pipérizino, dans lequel les groupes aryle sont du phényle ou du naphtyle, chacun desdits substituants de phényle et benzyle étant un groupe alkyle en C₁-C₆, alcoxy en C₁-C₆, fluoro ou chloro, et n est le nombre entier 0, 1 ou 2;
(c) X est de l'oxygène ou un groupe -N(R₄)-, dans lequel R₄ est de l'hydrogène ou un groupe alkyle en C₁-C₆, cycloalkyle en C₃-C₇, allyle, vinyle, acyle en C₁-C₅, phényle, phényle mono- ou disubstitué, benzyle, benzyle monosubstitué, alcoxy(C₁-C₄)carbonylalkyle en C₁-C₆, méthacryloxyalkyle en C₁-C₆, acryloyloxyalkyle en C₁-C₆, phénylalkyle en C₁-C₆, naphtyle, bicycloalkyle en C₄-C₁₂, acyloxy en C₂-C₄ ou les groupes hétéroaromatiques non substitués ou substitués pyridyle, furannyle, benzofurann-2-yle, benzofurann-3-yle, thiényle, benzothién-2-yle, benzothién-3-yle, dibenzofurannyle, dibenzothiényle, carbazolyle, benzopyridyle et indolyle, chacun desdits substituants des groupes phényle, benzyle et hétéroaromatiques étant un groupe alkyle en C₁-C₆ ou alcoxy en C₁-C₆;
(d) Y est de l'hydrogène, un groupe -N(R₄)- ou un groupe -C(R₅)R₆-, dans lequel R₅ et R₆ sont chacun de l'hydrogène ou un groupe alkyle en C₁-C₆ ou cycloalkyle en C₃-C₇, à la condition que lorsque Y est un groupe -(C(R₅)R₆)-, X soit de l'oxygène; et
(e) B et B' sont chacun choisis dans le groupe comprenant:
(i) les groupes aryle non substitués, mono-, di- et trisubstitués, phényle et naphtyle;
(ii) les groupes hétéroaromatiques non substitués, mono- et disubstitués pyridyle, furannyle, benzofurann-2-yle, benzofurann-3-yle, thiényle, benzothién-2-yle, benzothién-3-yle, dibenzofurannyle, dibenzothiényle, carbazolyle, benzopyridyle, indoloyle et fluorényle, chacun desdits substituants de ces groupes aryle et hétéroaromatiques dans les parties (i) et (ii) étant choisi dans le groupe comprenant les groupes hydroxy, aryle, c'est-à-dire phényle et naphtyle, monoalcoxy(C₁-C₆)aryle, dialcoxy(C₁-C₆)aryle, mono-alkyl(C₁-C₆)aryle, dialkyl(C₁-C₆)aryle, chloroaryfe, fluoroaryle, cycloalkylaryle en C₃-C₇, cycloalkyle en C₃-C₇, cycloalkyloxy en C₃-C₇, cycloalkyloxy(C₃-C₇)alkyle en C₁-C₆, cycloalkyloxy-(C₃-C₇)alcoxy en C₁-C₆, arylalkyle en C₁-C₆, arylalcoxy en C₁-C₆, aryloxy, aryloxyalkyle en C₁-C₆, aryloxyalcoxy en C₁-C₆, mono- et dialkyl(C₁-C₆)arylalkyle en C₁-C₆, mono- et dialcoxy(C₁-C₆)arylalkyle en C₁-C₆, mono- et dialkyl(C₁-C₆)arylalcoxy en C₁-C₆, mono- et dialcoxy(C₁-C₆)arylalcoxy en C₁-C₆, amino, monoalkylamino en C₁-C₆, dialkylamino en C₁-C₆, diarylamino, pipérazino, N-alkyl(C₁-C₆)pipérazino, N-arylpipérazino, aziridino, indolino, pipéridino, morpholino, thiomorpholino, tétrahydroquinoléino, tétra-hydroisoquinoléino, pyrryle, alkyle en C₁-C₆, chloroalkyle en C₁-C₆, fluoroalkyle en C₁-C₆, alcoxy en C₁-C₆, monoalcoxy(C₁-C₆)alkyle en C₁-C₄, acryloxy, méthacryloxy, bromo, chloro et fluoro;
(iii) les groupes représentés par les formules graphiques suivantes : 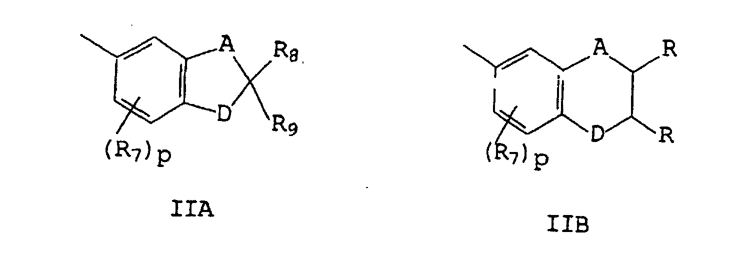 dans lesquelles A peut être du carbone ou de l'oxygène et D peut être de l'oxygène ou de l'azote substitué, pour autant que lorsque D est de l'azote substitué, A soit du carbone, lesdits substituants de l'azote étant choisis dans le groupe comprenant l'hydrogène et les groupes alkyle en C₁-C₆ et acyle en C₂-C₆, chaque R₇ est un groupe alkyle en C₁-C₆, alcoxy en C₁-C₆, hydroxy, chloro ou fluoro, R₈ et R₉ sont chacun de l'hydrogène ou un groupe alkyle en C₁-C₆, et p est le nombre entier 0, 1 ou 2;
(iv) les groupes alkyle en C₁-C₆, chloroalkyle en C₁-C₆, fluoroalkyle en C₁-C₆, alcoxy(C₁-C₆)alkyle en C₁-C₄, cycloakyle en C₃-C₆, mono-alcoxy(C₁-C₆)cycloakyle en C₃-C₆, monoalkyl(C₁-C₆)cycloakyle en C₃-C₆, chlorocycloalkyle en C₃-C₆, fluorocycloalkyle en C₃-C₆ et bicycloalkyle en C₄-C₁₂; et
(v) le groupe représenté par la formule graphique suivante : 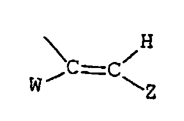 dans laquelle W peut être de l'hydrogène ou un groupe alkyle en C₁-C₄ et Z peut être choisi dans le groupe comprenant les membres non substitués, mono- et disubstitués du groupe comprenant les groupes naphtyle, phényle, furannyle et thiényle, chacun des substituants de ces groupes dans cette partie (v) étant un groupe alkyle en C₁-C₄, alcoxy en C₁-C₄, fluoro ou chloro; ou bien
(vi) B et B' pris ensemble forment un groupe fluorén-9-ylidène, fluorén-9-ylidène mono- ou disubstitué ou forment un membre choisi dans le groupe comprenant les cycles d'hydrocarbures spiro-monocycliques en C₃-C₁₂ saturés, les cycles d'hydrocarbures spiro-bicycliques en C₇-C₁₂ saturés et les cycles d'hydrocarbures spiro-tricycliques en C₇-C₁₂ saturés, chacun desdits substituants de fluorén-9-ylidène étant choisi dans le groupe comprenant les groupes alkyle en C₁-C₄, alcoxy en C₁-C₄, fluoro et chloro.

2. Naphtopyranne suivant la revendication 1, dans lequel:
(a) R₁ est de l'hydrogène et R₂ est de l'hydrogène ou un groupe alkyle en C₁-C₅, cycloalkyle en C₃-C₆, phényle, phényle mono- ou disubstitué, benzyle ou benzyle monosubstitué, chacun desdits substituants des groupes phényle et benzyle étant un groupe alkyle en C₁-C₄ ou alcoxy en C₁-C₄;
(b) R₃ est choisi dans le groupe comprenant les groupes alkyle en C₁-C₄, alcoxy en C₁-C₄, fluoro, phényle et aryloxy, et n est le nombre entier 0, 1 ou 2;
(c) X est de l'oxygène ou un groupe -N(R₄)-, dans lequel R₄ est de l'hydrogène ou un groupe alkyle en C₁-C₃, méthacryloxyalkyle en C₁-C₆ ou acryloxyalkyle en C₁-C₆;
(d) Y est de l'hydrogène, -NH- ou -CH₂-; et
(e) B et B' sont chacun choisis dans le groupe comprenant :
(i) les groupes phényle, phényle monosubstitués et phényle disubstitués;
(ii) les groupes hétéroaromatiques non substitués, mono- et disubstitués furannyle, benzofurann-2-yle, thiényle, benzothién-2-yle, dibenzofurannyle, aryloxy et diarylamino, chacun desdits substituants de ces groupes phényle et hétéroaromatiques étant choisi dans le groupe comprenant les groupes dialkylamino en C₁-C₃, pipéridino, morpholino, pyrryle, afkyle en C₁-C₃, chloroalkyle en C₁-C₃, fluoroalkyle en C₁-C₃, alcoxy en C₁-C₃, monoalcoxy(C₁-C₃)alkyle en C₁-C₃, fluoro et chloro;
(iii) les groupes représentés par les formules graphiques IIA et IIB, dans lesquelles A est du carbone et D est de l'oxygène, R₇ est un groupe alkyle en C₁-C₃ ou alcoxy en C₁-C₃, R₈ et R₉ sont chacun de l'hydrogène ou un groupe alkyle en C₁-C₄, et p est le nombre entier 0 ou 1;
(iv) les groupes alkyle en C₁-C₄; et
(v) le groupe représenté par la formule IIC dans laquelle W est de l'hydrogène ou du méthyle et Z est un groupe phényle ou phényle monosubstitué, ledit substituant de phényle étant choisi dans le groupe comprenant les groupes alkyle en C₁-C₃, alcoxy en C₁-C₃ et fluoro; ou bien
(vi) B et B' pris ensemble forment un groupe fluorén-9-ylidène, fluorén-9-ylidène monosubstitué ou un membre choisi dans le groupe comprenant les cycles d'hydrocarbures spiro-monocycliques en C₃-C₈ saturés, les cycles d'hydrocarbures spiro-bicycliques en C₇-C₁₀ saturés et les cycles d'hydrocarbure spiro-tricycliques en C₇-C₁₀ saturés, ledit substituant de fluorén-9-ylidène étant choisi dans le groupe comprenant les groupes alkyle en C₁-C₃, alcoxy en C₁-C₃, fluoro et chloro.

3. Naphtopyranne suivant la revendication 2, dans lequel:
(a) R₁ est de l'hydrogène et R₂ est de l'hydrogène ou un groupe alkyle en C₁-C₅ ou phényle;
(b) R₃ est choisi dans le groupe comprenant les groupes alkyle en C₁-C₃, alcoxy en C₁-C₃, phényle et aryloxy et n est le nombre entier 0, 1 ou 2;
(c) B et B' sont chacun choisis dans le groupe comprenant:
(i) les groupes phényle, phényle mono- et disubstitués;
(ii) les groupes hétéroaromatiques non substitués, mono- et disubstitués furannyle, benzofurann-2-yle, thiényle, benzothién-2-yle, dibenzofurannyle, aryloxy et diarylamino, chacun desdits substituants de ces groupes phényle et hétéroaromatiques étant choisi dans le groupe comprenant les groupes alkyle en C₁-C₃, alcoxy en C₁-C₃ et fluoro; et
(iii) le groupe représenté par la formule graphique IIA, dans laquelle A est du carbone et D est de l'oxygène, R₇ est un groupe alkyle en C₁-C₃ ou alcoxy en C₁-C₃, R₈ et R₉ sont chacun de l'hydrogène ou un groupe alkyle en C₁-C₃, et p est le nombre entier 0 ou 1; ou bien
(iv) B et B' pris ensemble forment un groupe fluorén-9-ylidène, adamantylidène, bornylidène, norbornylidène ou bicyclo-[3.3.1]nonan-9-ylidène.

4. Composé de naphtopyranne choisi dans le groupe comprenant :
a) le 7,7-diphényl-4-oxo-4H-7H-[1,3]dioxino[5',4':3,4]naphto[1,2-b]-pyranne;
b) le 7,7-di(4-méthoxyphényl)-4-oxo-4H-7H-[1,3]dioxino[5',4':3,4]-naphto[1,2-b]pyranne;
c) le 7-(4-méthoxyphényl)-7-phényl-2-pentyl-4-oxo-4H-7H-[1,3]dioxino-[5',4':3,4]naphto[1,2-b]pyranne;
d) le 7,7-diphényl-2-éthyl-4-oxo-4H-7H-[1,3]dioxino[5',4':3,4]naphto-[1,2-b]pyranne;
e) le 7,7-diphényl-2-pentyl-4-oxo-4H-7H-[1,3]dioxino[5',4':3,4]naphto-[1,2-b]pyranne;
f) le 7,7-diphényl-2-(2-méthylpropyl)-4-oxo-4H-7H-[1,3]dioxino-[5',4';3,4]naphto[1 ,2-b]pyranne;
g) le 2,7,7-triphényl-4-oxo-4H-7H-[1,3]dioxino[5',4':3,4]naphto[1,2-b]-pyranne;
h) le 7,7-diphényl-2-(1-phényléthyl)-4-oxo-4H-7H-[1,3]dioxino[5',4':3,4]-naphte[1,2-b]pyranne;
i) le 3-méthyl-7,7-diphényl-2,4-dioxo-2,3,4,7-tétrahydro[1,3]oxazino-[5',6':3,4]naphto[1,2-b]pyranne;
j) le 3-(2-éthoxycarboxyléthyl)-7,7-diphényl-2,4-dioxo-2,3,4,7-tétra-hydro[1,3]oxazino[5',6':3,4]naphto[1,2-b]pyranne;
k) le 3-hexyl-7,7-diphényl-2,4-dioxo-2,3,4,7-tétrahydro[1,3]oxazino-[5',6':3,4]naphte[1,2-b]pyranne;
l) le 3-(2-méthacryloyloxyéthyl)-7,7-diphényl-2,4-dioxo-2,3,4,7-tétra-hydro[1,3]oxazino[5',6':3,4]naphto[1,2-b]pyranne;
m) le 2,2,7,7-tétraphényl-4-oxo-2,3,4,7-tétrahydro-1H-pyrimido-[5',4':3,4]naphto[1,2-b]pyranne;
n) le 2,2,7,7-tétraphényl-4-oxo-2,3,4,7-tétrahydro-1H-[1,3]oxazino-[5',4':3,4]naphto[1,2-b]pyranne; et
o) le 7,7-diphényl-1,2,4,7-tétrahydro-2,2-diméthylpyranno[3',4':3,4]-naphto[1,2-b]pyranne.

5. Article photochromique comprenant une matière hôte organique polymérique et une quantité photochromique du composé de naphtopyranne de l'une quelconque des revendications 1 à 4.

6. Article photochromique comprenant, en combinaison, une matière hôte organique polymérique transparente solide et une quantité photochromique de chacun (a) d'au moins un composé de naphtopyranne de l'une quelconque des revendications 1 à 4 et (b) d'au moins un autre composé photochromique organique ayant au moins un maximum d'absorption activé dans la gamme entre environ 400 et 700 nanomètres.

7. Article photochromique suivant la revendication 6, dans lequel le composé photochromique organique (b) est choisi dans le groupe comprenant d'autres naphtopyrannes, les chromènes, les oxazines, les dithizonates métalliques, les fulgides et les fulgimides.

8. Article photochromique suivant l'une quelconque des revendications 5 à 7, dans lequel la quantité totale de composé photochromique présent est de 0,05 à 1,0 mg par centimètre carré de surface de matière hôte organique à laquelle la ou les substances photochromiques sont incorporées ou appliquées.

9. Article photochromique suivant l'une quelconque des revendications 5 à 7, dans lequel la matière hôte organique polymérique est choisie dans le groupe comprenant les poly(méthacrylates d'alkyle en C₁-C₁₂), les poly(diméthacrylates d'oxyalkylène), les poly(méthacrylates phénoliques alcoxylés), l'acétate de cellulose, le triacétate de cellulose, l'acétopropionate de cellulose, l'acétobutyrate de cellulose, le poly(acétate de vinyle), le poly(alcool vinylique), le poly(chlorure de vinyle), le poly(chlorure de vinylidène), les polycarbonates thermoplastiques, les polyesters, les polyuréthannes, le poly(éthylène téréphtalate), le polystyrène, le poly(alpha-méthylstyrène), le copoly(styrène-méthacrylate de méthyle), le copoly(styrène-acrylonitrile), le polyvinylbutyral et les polymères des membres du groupe comprenant les monomères de polyol(allyl carbonate), les monomères d'acrylate polyfonctionnels, les monomères de méthacrylate polyfonctionnels, les monomères de diéthylène glycol diméthacrylate, les monomères de diisopropényl benzène, les monomères de diméthacrylate de bisphénol A éthoxylé, les monomères d'éthylène glycol bisméthacrylate, les monomères de poly(éthylène glycol) bisméthacrylate, les monomères de bisméthacrylate de phénol éthoxylé, les monomères d'acrylate d'alcool polyatomique alcoxylé, les monomères de styrène, les monomères d'uréthanne acrylate, les monomères d'acrylate de glycidyle, les monomères de méthacrylate de glycidyle et les monomères de diallylidène pentaérythritol.

10. Article photochromique suivant la revendication 9, dans lequel la matière hôte organique polymérique est un polymère transparent solide choisi dans le groupe comprenant le poly(méthacrylate de méthyle), le poly(éthylène glycol bisméthacrylate), le poly(diméthacrylate de bisphénol A éthoxylé), les polycarbonates thermoplastiques, le poly(acétate de vinyle), le polyvinylbutyral, les polyuréthannes et les polymères des membres du groupe comprenant les monomères de diéthylène glycol bis(allyl carbonate), les monomères de diéthylène glycol diméthacrylate, les monomères de bisméthacrylate de phénol éthoxylé, les monomères de diisopropényl benzène et les monomères de triméthylol propane triacrylate éthoxylé.

11. Article photochromique suivant l'une quelconque des revendications 5 à 7, dans lequel la matière hôte est un polymérisat d'un monomère de résine organique optique.

12. Article photochromique suivant la revendication 11, dans lequel l'indice de réfraction du polymérisat est d'environ 1,48 à environ 1,75.

13. Article photochromique suivant la revendication 12, dans lequel l'indice de réfraction du polymérisat est d'environ 1,495 à environ 1,66.

14. Article photochromique suivant l'une quelconque des revendications 5 à 13, dans lequel ledit article est un élément optique.

15. Article photochromique suivant la revendication 14, dans lequel ledit élément optique est une lentille.
